(19) Europäisches Patentamt
European Patent Office
Office européen des brevets

(11) Veröffentlichungsnummer: **0 130 151**
**A1**

(12) # EUROPÄISCHE PATENTANMELDUNG

(21) Anmeldenummer: 84810305.7

(22) Anmeldetag: 21.06.84

(51) Int. Cl.4: C 07 D 405/14
A 61 K 31/41, A 61 K 31/415

(30) Priorität: 27.06.83 CH 3498/83

(43) Veröffentlichungstag der Anmeldung: 02.01.85 Patentblatt 85/1

(84) Benannte Vertragsstaaten: AT BE CH DE FR GB IT LI LU NL SE

(71) Anmelder: CIBA-GEIGY AG
Postfach
CH-4002 Basel(CH)

(72) Erfinder: Hubele, Adolf, Dr.
Obere Egg 9
CH-4312 Magden(CH)

(72) Erfinder: Riebli, Peter
Bünten 17
CH-4446 Buckten(CH)

(54) **Neue 2-substituierte Benzofuranderivate.**

(57) Die Erfindung betrifft neue 2-substituierte Benzofuranderivate der Formel I

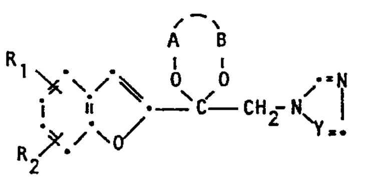

(I)

sowie Salze davon, Verfahren zu ihrer Herstellung, diese enthaltende pharmazeutische Präparate und ihre Verwendung als Arzneimittel oder agrarchemische Mittel.

EP 0 130 151 A1

CIBA-GEIGY AG    4-14482/+

Basel (Schweiz)

Neue 2-substituierte Benzofuranderivate.

Die Erfindung betrifft 2-substituierte Benzofuranderivate der Formel I

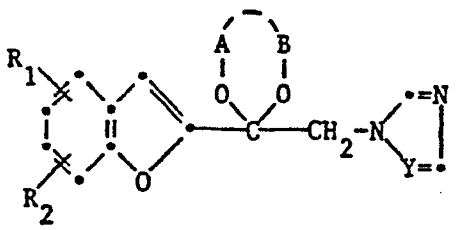 (I) ,

worin

Y für -CH= oder -N= steht:

$R_1$ und $R_2$ unabhängig voneinander für Wasserstoff, Halogen, $C_1$-$C_4$-Alkyl oder $C_1$-$C_4$-Alkoxy stehen;

A und B für $C_1$-$C_{12}$-Alkyl stehen oder zusammen eine der folgenden Alkylenbrücken

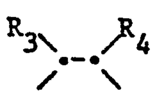 (Ia), 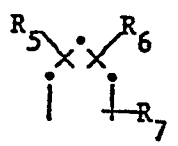 (Ib) oder 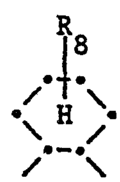 (Ic)

bedeuten, wobei

$R_3$ und $R_4$ unabhängig voneinander für Wasserstoff, ein durch Halogen oder eine Gruppe der Formel $-Z-R_9$ substituiertes oder unsubstituiertes $C_1$-$C_{12}$-Alkyl oder für durch Halogen, $C_1$-$C_4$-Alkoxy und/oder $C_1$-$C_4$-Alkyl substituiertes oder unsubstituiertes Phenyl stehen, wobei Z Sauerstoff oder Schwefel bedeutet und

$R_9$ Wasserstoff, ein durch $C_1$-$C_4$-Alkoxy substituiertes oder unsubstituiertes $C_1$-$C_8$-Alkyl, $C_3$-$C_4$-Alkenyl, 2-Propinyl oder 3-Halogen-2-propinyl, ein durch Halogen, $C_1$-$C_4$-Alkyl, $C_1$-$C_4$-Alkoxy, Nitro, und/oder Trifluormethyl substituiertes oder unsubstituiertes Phenyl oder ein durch Halogen, $C_1$-$C_4$-Alkyl und/oder $C_1$-$C_4$-Alkoxy substituiertes oder unsub-

stituiertes Benzyl bedeutet;

$R_5$, $R_6$ und $R_7$ unabhängig voneinander Wasserstoff oder $C_1$-$C_4$-Alkyl bedeuten, wobei die Gesamtzahl der Kohlenstoffatome in $R_5$, $R_6$ und $R_7$ die Zahl 6 nicht übersteigen soll, und

$R_8$ für Wasserstoff oder $C_1$-$C_3$-Alkyl steht,

und ihrer Salze.

Unter Alkyl oder Alkylanteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, Undecyl oder Dodecyl sowie ihre Isomeren, wie z.B. Isopropyl, Isobutyl, tert.-Butyl, Isopentyl usw. Alkenyl steht z.B. für Propenyl-(1), Allyl, Butenyl-(1), Butenyl-(2), Butenyl-(3) usw. Unter Halogen soll hier und im folgenden Fluor, Chlor, Brom oder Jod, vorzugsweise Chlor oder Brom verstanden werden.

Die Erfindung betrifft sowohl die freien Verbindungen der Formel I, als auch deren Additionssalze mit anorganischen und organischen Säuren.

Erfindungsgemässe Salze sind insbesondere Additionssalze mit pharmakologische unbedenklichen anorganischen oder organischen Säuren.

Physiologisch unbedenkliche Säuren, d.h. pharmazeutisch verwendbare Säuren, sind beispielsweise pharmazeutisch verwendbare Mineralsäuren, wie Halogenwasserstoffsäuren, z.B. Chlor-, Brom- oder Jodwasserstoffsäure, Schwefelsäure oder Phosphorsäure, pharmazeutisch verwendbare Carbon- und Sulfonsäuren, wie aliphatische Mono- und gegebenenfalls hydroxylierte Dicarbonsäuren, z.B. Essigsäure, Fumarsäure, Maleinsäure, Apfelsäure oder Weinsäure, ebenso aliphatische oder aromatische Sulfonsäuren, wie Niederalkan- oder gegebenenfalls substituierte Benzolsulfonsäure, z.B. Methan-, Ethan-, Benzol-, p-Toluol- und p-Brombenzolsulfonsäure, ferner Sulfaminsäuren, z.B. N-Cyclohexylsulfaminsäure.

Die Verbindungen der Formel I und ihre Salze sind biologisch aktive Substanzen, die sich zur Beeinflussung des Pflanzenwachstums sowie zur Bekämpfung schädlicher, insbesondere phytopathoger Mikroorganismen, einsetzen lassen, vor allem aber vorteilhafte pharmakologische, insbesondere antimykotische Eigenschaften sowie vorteilhafte Wirkungen auf das zentrale Nervensystem, insbesondere antikonvulsive, anxiolytische und/oder antidepressive Eigenschaften, aufweisen. Sie lassen sich dementsprechend als antimykotische und/oder antikonvulsive, anxiolytische und/oder antidepressive Arzneimittelwirkstoffe, beispielsweise zur Bekämpfung von Mykosen, d.h. parasitären Pilzen sowie zur Behandlung von Epilepsie, Spannungs-, Angst- und Depressionszuständen einsetzen. Die Verbindungen der Formel I sind auch, verglichen mit anderen antikonvulsionen bzw. antidepressiven Arzneimittelwirkstoffen, sehr gut verträglich. So ist die $LD_{50}$ an Maus und Ratte durchwegs deutlich hoher als 1000 mg/kg p.o. Auch wurde an den genannten Kleinnagerspezies bis zur Dosierung von 200 mg/kg p.o. keinerlei Störung der Motorik festgestellt.

Die die Verwendbarkeit der Verbindungen der Formel I und ihrer pharmazeutisch verwendbaren Säureadditionssalze zur Bekämpfung an Warmblütern parasitierender Pilze begründenden antimycotischen Eigenschaften können beispielsweise in vitro mittels üblicher mikrobiologischer Untersuchungsverfahren, z.B. anhand ihrer toxischen Wirkung auf an Warmblütern parasitierende Pilzstämme, wie Trychophyton mentagrophytes, Microsporum canis, Sporotrichum schenkii, Aspergillus fumigatus und Candida albicans, ebenso in vivo am Meerschweinchen anhand der Heilwirkung auf experimentelle Infestationen der Rückenhaut mit Trychophyton, z.B. T. rubrum, nach peroraler bzw. lokaler Applikation, nachgewiesen werden.

Die antikonvulsive Wirksamkeit der Verbindungen der Formel I und ihrer pharmazeutisch verwendbaren Säureadditionssalze kann <u>in vivo</u> beispielsweise an der Maus und an der Ratte im Elektroschock-Test im Dosis-Bereich von 10 bis 100 mg/kg p.o. demonstriert werden, ausserdem an der Maus im Pentatetrazolkrampf-Test im ähnlichen Dosisbereich. Die anxiolytische Wirksamkeit lässt sich an der Maus und an anderen Kleinnagern anhand des Quatre-Plaques-Testes im Dosisbereich von etwa 10 bis 100 mg/kg p.o. zeigen. Die Substanzen beeinflussen auch Benzodiazepin-Rezeptoren <u>in vivo</u> (Chang und Snyder, Eur.J.Pharmac. <u>48</u>, (1978), 213-218. Ausserdem wirken sie bereits bei Dosen unter 30 mg/kg p.o. stimulierend auf die Motilität der Maus und wirken antagonistisch auf Temperatur-Effekte des Apomorphins. Aus dem genannten Wirkungsprofil lassen sich antikonvulsive, anxiolytische sowie antidepressive Wirkungen ableiten.

Bevorzugt aufgrund ihrer guten antimykotischen als auch sehr guten antikonvulsiven, anxiolytischen und/oder antidepressiven Wirkung sind Verbindungen der Formel I, worin $R_1$ und $R_2$ unabhängig voneinander Wasserstoff, $C_1$-$C_4$-Alkyl, wie Methyl, $C_1$-$C_4$-Alkoxy, wie Methoxy, oder Halogen der Atomnummer bis und mit 35, wie Chlor, bedeuten, A und B unabhängig voneinander $C_1$-$C_4$-Alkyl, wie Methyl, darstellen oder gemeinsam eine Alkylenbrücke der Formeln Ia oder Ib

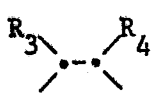 (Ia) 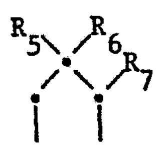 (Ib)

bilden, in der $R_3$ Wasserstoff, $C_1$-$C_4$-Alkyl, wie Methyl, oder Hydroxy-$C_1$-$C_4$-alkyl, wie Hydroxymethyl, und $R_4$ Wasserstoff, $C_1$-$C_4$-Alkyl, wie Methyl oder Ethyl, $C_1$-$C_4$-Alkoxy-$C_1$-$C_4$-alkyl, wie Methoxy- oder Ethoxymethyl, oder unsubstituiertes oder im Phenyl durch $C_1$-$C_4$-Alkyl, wie Methyl, $C_1$-$C_4$-Alkoxy, wie Methoxy, oder Halogen mit Atomnummer bis und mit 35, wie Chlor, substituiertes Phenoxy-$C_1$-$C_4$-

alkyl, wie Phenoxymethyl, bedeutet bzw. $R_5$, $R_6$ und $R_7$ unabhängig voneinander Wasserstoff, $C_1$-$C_4$-Alkyl, wie Methyl, Ethyl, Propyl oder Butyl, darstellen, und Y eine Gruppe -CH= oder -N= bedeutet, insbesondere solche, worin $R_1$ und $R_2$ entweder Wasserstoff oder Halogen der Atomnummer bis und mit 35, wie Chlor, welches insbesondere in 5- und 7-Stellung gebunden ist, bedeuten, sowie ihre Salze, insbesondere pharmazeutisch verwendbaren Salze.

Aufgrund ihrer antimykotischen und/oder antikonvulsiven, anxiolytischen bzw. antidepressiven Wirkung sind vor allem bevorzugt:

2-(Benzofuran-2-yl)-2-[1-(1H-1,2,4-triazolyl)-methyl]-1,3-dioxolan,
2-(Benzofuran-2-yl)-2-[1-(1H-1,2,4-triazolyl)-methyl]-5-ethyl-5-methyl-1,3-dioxan,
2-(Benzofuran-2-yl)-2-[1-(1H-1,2,4-triazolyl)-methyl]-5,5-dimethyl-4-propyl-1,3-dioxan,
2-(Benzofuran-2-yl)-2-[1-(1H-1,2,4-triazolyl)-methyl]-5,5-diethyl-1,3-dioxan,
2-(Benzofuran-2-yl)-2-[1-(1H-1,2,4-triazolyl)-methyl]-4-methyl-1,3-dioxan,
2-(Benzofuran-2-yl)-2-[1-(1H-1,2,4-triazolyl)-methyl]-5-ethyl-5-butyl-1,3-dioxan,
2-(Benzofuran-2-yl)-2-[1-(1H-1,2,4-triazolyl)-methyl]-5-ethyl-4-propyl-1,3-dioxan,
2-(Benzofuran-2-yl)-2-[1-(1H-1,2,4-triazolyl)-methyl]-1,3-dioxan,
2-(Benzofuran-2-yl)-2-[1-(1H-1,2,4-triazolyl)-methyl]-4-methoxymethyl-1,3-dioxolan,
2-(Benzofuran-2-yl)-2-[1-(1H-1,2,4-triazolyl)-methyl]-4-ethoxymethyl-1,3-dioxolan,
2-(Benzofuran-2-yl)-2-[1-(1H-1,2,4-triazolyl)-methyl]-4,5-dimethyl-1,3-dioxolan
2-(Benzofuran-2-yl)-2-[1-(1H-1,2,4-triazolyl)-methyl]-4-phenoxymethyl-1,3-dioxolan,

2-(Benzofuran-2-yl)-2-(1-imidazolyl-methyl)-1.3-dioxolan,
2-(Benzofuran-2-yl)-2-(1-imidazolyl-methyl)-5,5-dimethyl-1,3-dioxan,
2-(Benzofuran-2-yl)-2-(1-imidazolyl-methyl)-4-methoxymethyl-1,3-dioxolan,
2-(5,7-Dichlor-benzofuran-2-yl)-2-[1-(1H-1,2,4-triazolyl)-methyl]-4-ethyl-1,3-dioxolan,
2-(5,7-Dichlor-benzofuran-2-yl)-2-[1-(1H-1,2,4-triazolyl)-methyl]-1,3-dioxolan,
2-(5,7-Dichlor-benzofuran-2-yl)-2-[1-(1H-1,2,4-triazolyl)-methyl]-4-methyl-1,3-dioxolan,
2-(5,7-Dichlor-benzofuran-2-yl)-2-[1-(1H-1,2,4-triazolyl)-methyl]-5,5-dimethyl-1,3-dioxan,
2-(5,7-Dichlor-benzofuran-2-yl)-2-[1-(1H-1,2,4-triazolyl)-methyl]-4-ethoxymethyl-1,3-dioxolan,
2-(5,7-Dichlor-benzofuran-2-yl)-2-[1-(1H-1,2,4-triazolyl)-methyl]-4-methoxymethyl-1,3-dioxolan,
2-(5,7-Dichlor-benzofuran-2-yl)-2-[1-(1H-1,2,4-triazolyl)-methyl]-5-methyl-5-propyl-1,3-dioxan,
2-(5,7-Dichlor-benzofuran-2-yl)-2-(1-imidazolylmethyl)-1,3-dioxolan,
2-(5,7-Dichlor-benzofuran-2-yl)-2-(1-imidazolylmethyl)-4-methoxymethyl-1,3-dioxolan,
2-(5,7-Dichlor-benzofuran-2-yl)-2-(1-imidazolylmethyl)-4-ethoxymethyl-1,3-dioxolan und
1-(Benzofuran-2-yl)-1,1-dimethoxy-2-[1-(1H-1,2,4-triazolyl)]-ethan,

sowie Salze, insbesondere pharmazeutisch verwendbare Säureadditionssalze davon. Die Erfindung betrifft ferner ein auf an sich bekannten Methoden beruhendes Verfahren zur Herstellung von Verbindungen der Formel I und ihrer Salze.

Dieses ist dadurch gekennzeichnet, dass indem man

A) eine Verbindung der Formel II

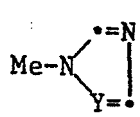 (II),

worin Me Wasserstoff oder ein Metallkation darstellt, mit einer Verbindung der Formel III

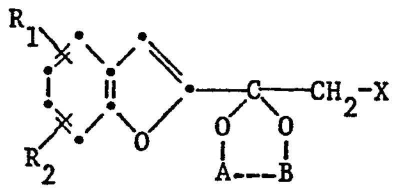

(III),

worin X eine nukleofuge Abgangsgruppe bedeutet, kondensiert oder

B) in einer Verbindung der Formel IV

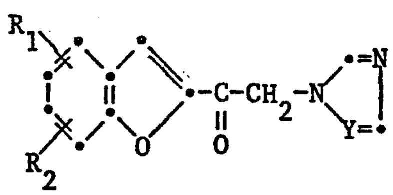

(IV),

die Carbonylgruppe in eine Gruppe der Formel V

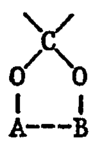

(V)

überführt, oder

C) zur Herstellung von Verbindungen der Formel I, worin A und B gemeinsam eine Gruppe der Formel $-CH_2-CH(CH_2ZR_9)-$ darstellen und $R_9$ einen von Wasserstoff verschiedenen Rest $R_9$ bedeutet, Verbindungen der Formel VI und VII

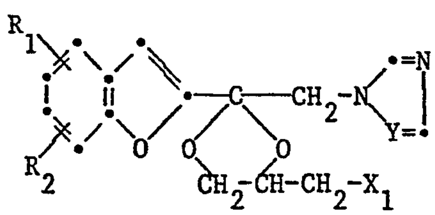

(VI) und $X_2 - R_9$ (VII),

worin einer der Reste $X_1$ und $X_2$ gegebenenfalls in Salzform vorliegendes Hydroxy oder Mercapto, z.B. der Formel -Z-Me, und der andere eine nukleofuge Abgangsgruppe X bedeutet oder sowohl $X_1$ als auch $X_2$ Hydroxygruppen darstellen, miteinander kondensiert oder

D) eine Verbindung der Formel

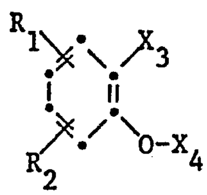

(VIII),

worin einer der Reste $X_3$ und $X_4$ eine Gruppe der Formel

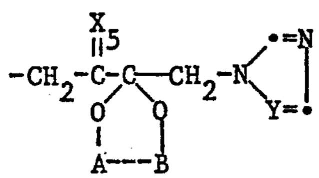

(VIIIa),

in der $X_5$ eine gegebenenfalls funktionell abgewandelte Oxogruppe ist, und der andere Wasserstoff oder $X_3$ eine Gruppe der Formel

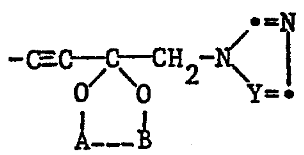

(VIIIb)

und $X_4$ Wasserstoff bedeutet, cyclisiert oder

E) eine Verbindung der Formel

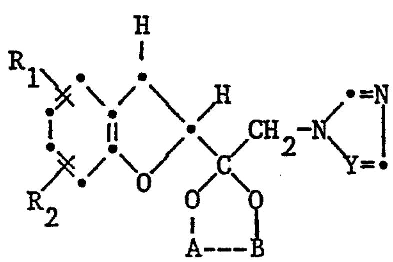

(IX)

dehydriert und, wenn erwünscht, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

Metallkationen Me sind dabei beispielsweise Alkalimetall-, z.B. Lithium-, Natrium- oder Kaliumkationen,oder erdalkalimetallische Gruppen der Formel $[M^{II}/2]^+$ bzw. $[M^{II}-Hal]^+$, worin $M^{II}$ ein Erdalkalimetall-, z.B. Calcium- oder Magnesiumatom und Hal ein Halogen, z.B. Chlor, Brom oder Jod,bedeutet.

Nukleofuge Abgangsgruppen X sind beispielsweise reaktionsfähige verestere Hydroxygruppen, wie mit einer Halogenwasserstoffsäure, z.B. mit Chlor-, Brom- oder Jodwasserstoffsäure, oder einer Niederalkan-, gegebenenfalls substituierten Benzol- oder Halogensulfonsäure, z.B. mit Methan-, Ethan-, Ethen-, Benzol-, p-Toluol- oder Fluorsulfonsäure, veresterte Hydroxygruppen.

Die Umsetzung eines Azols der Formel II, worin Me bevorzugt ein Metallatom, insbesondere ein Alkalimetallatom darstellt, mit einer Verbindung der Formel III, worin X beispielsweise für Halogen, insbesondere Chlor, Brom oder Jod, oder Benzolsulfonyloxy, p-Tosyloxy, Trifluoracetyloxy oder bevorzugt Niederalkylsulfonyloxy wie z.B. Mesyloxy, bedeutet, wird bevorzugt in einem relativ polaren, jedoch reaktionsinerten organischen Lösungsmittel durchgeführt, z.B. N,N-Dimethylformamid, N,N-Dimethylacetamid, Dimethylsulfoxid, Acetonitril, Benzonitril und anderen. Derartige Lösungsmittel können in Kombination mit anderen reaktionsinerten Lösungsmitteln, wie aliphatischen oder aromatischen Kohlenwasserstoffen, z.B. Benzol, Toluol, Xylol, Hexan, Petrolether, Chlorbenzol, Nitrobenzol u.a. verwendet werden.

Bedeutet X Chlor oder Brom, so kann man zweckmässig Alkalijodid (wie NaJ oder KJ) zur Beschleunigung der Reaktion zusetzen. Erhöhte Temperaturen von 0 bis 220°C, bevorzugt 80-170°C, sind vorteilhaft. Zweckmässig wird das Reaktionsgemisch unter Rückfluss erhitzt.

In den Fällen, in denen in Formel II Me für Wasserstoff steht, wird das Verfahren in Gegenwart einer Base durchgeführt. Beispiele solcher Basen sind anorganische Basen wie die Oxide, Hydroxide, Hydride, Carbonate und Hydrogencarbonate von Alkali und Erdalkalimetallen sowie z.B. tert. Amine wie Triethylamin, Triethylendiamin, Pyridin, 4-Dimethylaminopyridin, 4-Pyrrolidylpyridin usw.

Bei dieser und den folgenden Herstellungsvarianten können die Zwischen- und Endprodukte aus dem Reaktionsmedium isoliert und, falls erwünscht, auf eine der allgemein üblichen Methoden gereinigt werden, z.B. durch Extraktion, Kristallisation, Chromatographie, Destillation usw..

Die Ueberführung der Carbonylgruppe in Verbindungen der Formel IV in die Gruppe der Formel V erfolgt durch Umsetzung mit einem Orthocarbonsäure-tri-$C_1$-$C_{12}$-alkylester oder in Gegenwart einer Säure mit mindestens 2 Mol eines einwertigen Alkohols der Formel A-OH (Va), wobei Verbindungen der Formel I erhalten werden, worin A und B gleiche $C_1$-$C_{12}$-Alkylgruppen bedeuten, oder durch Umsetzung mit einem Diol der Formel Vb

HO-A------B-OH (Vb),

wobei Verbindungen der Formel I erhalten werden, worin A und B gemeinsam eine der eingangs definierten Alkylenbrücken darstellen.

Diese Ketalisierungsreaktion kann analog zu bereits bekannten Ketalisierungs-Reaktionen durchgeführt werden, beispielsweise analog zur Herstellung von 2-Brommethyl-2,4-diphenyl-1,3-dioxolan [Synthesis, 1974 (I), 23].

Bei der bevorzugten Ausführung der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z.B. Benzol, Toluol, Xylol, 1,1,1-Trichloräthan, Tri- oder Tetrachlormethan in Frage, wobei zur Beschleunigung der Reaktion ein Zusatz einer starken Säure, wie z.B. p-Toluolsulfonsäure vorteilhaft sein kann. Verwendbare organische Lösungsmittel sind in diesem Fall z.B. aromatische Kohlenwasserstoffe, wie Benzol, Toluol, Xylol usw. und gesättigte Kohlenwasserstoffe, wie n-Hexan.
Es sind auch weitere Wege der Ketalisierung durchführbar, z.B. indem man ein Keton IV, das mit einem von Alkanolen bzw. Diolen der Formeln Va bzw. Vb verschiedenem Alkohol, z.B. mit einem gegebenenfalls substituierten Phenol oder Brenzkatechin, ketalisiert ist, umsetzt und dieses durch Reaktion mit überschüssigem Alkanol Va bzw. dem Diol (Vb) zu (I) umketalisiert. Das Ausgangsprodukt ist z.B. gemäss einer der Verfahrensvarianten A) und D) zugänglich.

Die Herstellung von Verbindungen der Formel I, worin Substituenten A und B zusammen für $-CH_2-CH(CH_2ZR_9)-$ stehen, gemäss Variante C) erfolgt beispielsweise durch Reaktion von Verbindungen der Formeln VI und VII, worin $X_1$ eine Gruppe -ZH und $X_2$ eine Gruppe X bedeutet. Die Reaktion wird bevorzugt in reaktions-inerten organischen Lösungsmitteln durchgeführt. Es eignen sich hierzu z.B. N,N-Dimethylformamid, N,N-Dimethylacetamid, Hexamethylphosphortriamid, Dimethylsulfoxid, 4-Methyl-4-pentanon usw. Auch Gemische mit anderen reaktionsinerten Lösungsmitteln, z.B. mit aromatischen Kohlenwasserstoffen wie Benzol, Toluol, Xylol usw. können verwendet werden. In manchen Fällen kann es sich als vorteilhaft erweisen, zur Beschleunigung der Reaktionsgeschwindigkeit in Gegenwart einer Base zu arbeiten. Solche Basen sind z.B. Alkalimetallhydride oder Alkalimetallcarbonate. In gewissen Fällen kann es auch von Vorteil sein, dass man die Verbindung VI zuerst auf bekannte Art in ein geeignetes Metall-Salz überführt.

Dies geschieht vorzugsweise durch Reaktion von VI mit einer Na-Verbindung, z.B. Natriumhydrid, Natriumhydroxid usw.. Anschliessend wird dieses Salz von VI mit der Verbindung der Formel VII umgesetzt. Zur Erhöhung der Reaktionsgeschwindigkeit kann in manchen Fällen auch bei erhöhter Temperatur, vorzugsweise 80°C bis 130°C, bzw. am Siedepunkt des Lösungsmittels gearbeitet werden.

In analoger Weise kann man auch Verbindungen der Formeln VI und VII, worin $X_1$ eine Gruppe X und $X_2$ eine Gruppe -ZH ist, umsetzen.

Bei der zu Verbindungen der Formel I, worin Z Sauerstoff ist, führenden Kondensations-Reaktion von Verbindungen der Formeln VI und VII, worin $X_1$ und $X_2$ Hydroxy darstellen, können die Reaktanden in einem geeigneten Lösungsmittel unter Rückfluss erhitzt werden, wobei gleichzeitig das entstehende Wasser azeotrop aus dem Reaktionsgemisch abdestilliert wird. Als Lösungsmittel kommen aromatische Kohlenwasserstoffe, wie Toluol oder der Alkohol VII selbst in Frage. Bei dieser Reaktion arbeitet man zweckmässigerweise in Gegenwart einer starken Säure, z.B. p-Toluolsulfonsäure.

Die Cyclisierung von Verbindungen der Formel VIII gemäss Variante D) erfolgt in üblicher Weise, erforderlichenfalls in Gegenwart eines Kondensationsmittels, unter Kühlen oder Erwärmen, z.B. im Temperaturbereich von etwa 0° bis etwa 150°C, und/oder unter Inertgas, wie Stickstoff. Als Kondensationsmittel kommen beispielsweise saure Kondensationsmittel, wie Protonensäuren bzw. deren Anhydride oder sauren Salze, z.B. Mineralsäuren, wie Halogenwasserstoffsäuren, z.B. Chlor- oder Bromwasserstoffsäure, Schwefelsäure oder Phosphorsäure bzw. Polyphosphorsäure, vorteilhaft in Gegenwart eines gegenüber den Reaktionsteilnehmern inerten Lösungs- oder Verdünnungsmittels, in Betracht. So führt man die Cyclisierung von Verbindungen der Formel VIII, worin einer der Reste $X_3$ und $X_4$ eine Gruppe der Formel VIIIa und $X_5$ Oxo oder Thioxo darstellt, beispielsweise mittels Schwefelsäure oder Polyphosphorsäure durch, während man die Cyclisierung einer Verbindung der Formel VIII, worin $X_3$ eine Gruppe der Formel VIIIa und $X_5$ gegebenenfalls substituiertes Imino bedeutet, in einer essigsauren Lösung von Chlorwasserstoff durchführt. Die Cyclisierung von Verbindungen der Formel VIII, worin $X_3$ eine Gruppe der Formel VIIIb bedeutet, erfolgt spontan unter den Bedingungen ihrer Bildung, z.B. durch Stehenlassen, gegebenenfalls unter gelindem Erwärmen, in Pyridin.

Funktionell abgewandelte Oxogruppen $X_5$ sind dabei beispielsweise Thioxo- oder gegebenenfalls substituierte Iminogruppen, wie Diniederalkylimino oder gegebenenfalls substituiertes Anilo.

Die Dehydrierung von Verbindungen der Formel IX gemäss Variante E) erfolgt in üblicher Weise, beispielsweise durch Behandlung mit Schwefel oder Selen, mit Edelmetallkatalysatoren, z.B. Palladium auf Kohle, oder mittels N-Bromsuccinimid.

Verfahrensgemäss erhältliche Verbindungen können nach an sich bekannten Methoden in andere Verbindungen der Formel I überführt werden.

So kann man beispielsweise verfahrensgemäss erhältliche Verbindungen zu anderen Verbindungen der Formel I umketalisieren. Beispielsweise kann man in Verbindungen der Formel I, worin A und B gleiche $C_1$-$C_{12}$-Alkylreste A darstellen, durch Umsetzung mit 1 Mol eines anderen $C_1$-$C_{12}$-Alkanols der Formel B-OH (Vc), eine Gruppe A durch eine Gruppe B oder durch Umsetzung mit einem Diol der Formel Vb beide Gruppen A durch einen zweiwertigen Rest ersetzen. Die Umketalisierung erfolgt in üblicher Weise, beispielsweise in Gegenwart eines sauren Kondensationsmittels, wie einer Mineral-, Sulfon- oder starken Carbonsäure, z.B. von Chlor- oder Bromwasserstoffsäure, Schwefelsäure, p-Toluolsulfonsäure oder Trifluoressigsäure, vorteilhaft unter destillativer bzw. azeotrop-destillativer Entfernung leichtflüchtiger Reaktionsprodukte.

Ferner kann man in den carbocyclischen Arylteil verfahrensgemäss erhältlicher Verbindungen gegebenenfalls zusätzliche Substituenten $R_1$ und/oder $R_2$ einführen. So kann man z.B. durch Umsetzung mit einem Halogen in Gegenwart einer Lewissäure, wie einem Eisen-, Zink-, Bor- oder Antimonhalogenid, oder durch Behandlung mit N-Chlorsuccinimid, Halogen einführen.

Ferner kann man Halogen durch Umsetzung mit einer Alkylmetallverbindung, z.B. mit einem Alkyllithium oder Alkylmagnesiumhalogenid, durch Alkyl ersetzen.

Werden die Verbindungen der Formel I als Basen erhalten, dann lassen sie sich durch anorganische oder organische Säuren in entsprechende Salze oder durch vorzugsweise äquimolare Mengen von Metallsalzen in Metallkomplexe der Formel I überführen. Umgekehrt lassen sich Salze der Formel I beispielsweise durch Reaktion mit Alkali(hydrogen)carbonat oder Alkalihydroxid in die freien Basen der Formel I überführen.

Die Ausgangsketale der Formel III lassen sich aus Ketonen der Formel X

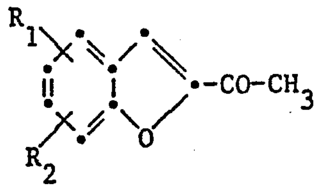

(X)

durch Reaktion mit dem gewünschten Alkanol, Diol oder Orthocarbonsäureester in einem inerten Lösungsmittel, z.B. einem halogenierten Kohlenwasserstoff, wie Methylenchlorid, Aethylenchlorid, Chloroform, Tetrachlorkohlenstoff usw., und gleichzeitige oder anschliessende Halogenierung, herstellen, wobei zur Beschleunigung der Reaktion ein Zusatz von p-Toluolsulfonsäure vorteilhaft ist. Zwischenprodukte der Formel IV werden z.B. hergestellt durch Umsetzung eines Ketons der Formel X mit Halogen und Weiterreaktion des Halogenketons der Formel XI

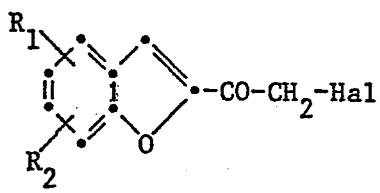

(XI)

mit einem Azol der Formel II analog zu Variante A. Dabei steht Hal bevorzugt für Chlor oder Brom.

Die Ketale III, VI, VIII und IX erhält man analog zu Variante b) durch Reaktion des Ausgangsketons z.B. der Formel IV mit einem geeigneten Alkohol oder Diol.

Die Zwischenprodukte der Formeln III, IV, VI, VIII und IX, die speziell für die Herstellung der erfindungsgemässen Verbindungen der Formel I entwickelt wurden, sind noch neu. Sie bilden, ebenso wie Verfahren zu ihrer Herstellung ebenfalls einen Gegenstand der Erfindung.

Die Verbindungen der Formel I können in stereoisomeren Formen, beispielsweise je nach Anzahl der asymmetrischen Kohlenstoffatome in Form eines individuellen Enantiomeren bzw. Diastereomeren oder als Gemische derselben vorliegen. So können bei den beschriebenen Ketalisierungs-Reaktionen eines Ketons mit einem substituierten Diol Gemische von Enantiomeren bzw. Diastereomeren des resultierenden Ketals entstehen. Zur Veranschaulichung eines Diastereomerenpaares von Verbindungen der Formel I soll das nachfolgende Beispiel dienen:

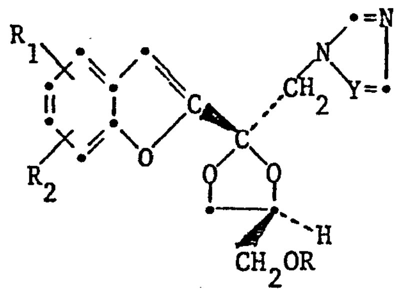

(Ia)

Die Konfiguration Ia soll hier und im folgenden als das "cis"-Isomere bezeichnet werden.

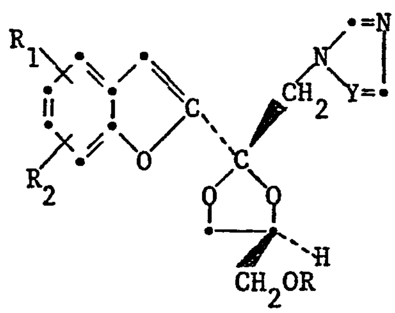

(Ib)

.... = hinter

—— = in

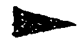 = vor der Zeichenebene

Die Konfiguration Ib soll entsprechend als das "trans"-Isomere bezeichnet werden. Die Charakterisierung der beiden Konfigurationen kann z.B. mit Hilfe der NMR-Spektroskopie oder der Röntgenstrukturanalyse erfolgen. Die vorliegende Erfindung bezieht sich auf sämtliche isomeren Verbindungen und ihre Salze. Die Trennung von Diastereomeren, z.B. von Ia und Ib, kann beispielsweise durch fraktionierte Kristallisation oder durch Chromatographie (Dünn-, Dickschicht-, Säulenchromatographie, Flüssigkeitshochdruckchromatographie usw.) erfolgen. Die beiden Isomeren zeigen unterschiedliche biologische Wirkung. Enantiomerenpaare können durch Kristallisation aus einem optisch aktiven Lösungsmittel, Chromatographie an einer optisch aktiven mobilen oder stationären Phase oder Bildung diastereomerer Hilfsverbindungen, z.B. Säureadditionssalze mit optisch aktiven Säuren, Trennung der Diastereomeren und Freisetzung des gewünschten Enantiomeren aufgespalten werden. Im allgemeinen werden für praktische Zwecke die Stereoisomerengemische verwendet.

Die Erfindung betrifft ebenfalls die Verwendung von Verbindungen der Formel I und ihrer pharmazeutisch verwendbaren Säureadditionssalze zur topischen bzw. lokalen und systemischen Bekämpfung an Warmblütern parasitierender Pilze bzw. zur systemischen Behandlung von Krampf- und Spannungszuständen, namentlich solcher der Epilepsie von Angst- und Depressionszuständen, insbesondere als Wirkstoff in bzw. zur Herstellung von pharmazeutischen Präparaten, sowie derartige pharmazeutische Präparate.

Bei den erfindungsgemässen pharmazeutischen Präparaten, welche Verbindungen der Formel I oder pharmazeutisch verwendbare Salze davon enthalten, handelt es sich dementsprechend um solche zur enteralen, wie oralen oder rektalen, und parenteralen Verabreichung sowie zur topischen bzw. lokalen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der zu behandelnden Krankheit der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von etwa 50-500 mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60% des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes

Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess-, Regulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat, oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Als topisch bzw. lokal anwendbare pharmazeutische Präparate kommen in erster Linie Creme, Salben, Pasten, Puder, Schäume, Tinkturen und Lösungen, in Frage, die von etwa 0,5 bis etwa 20% des Wirkstoffs enthalten.

Creme sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline

(Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukten davon, wie Polyglycerinfettsäureester, Polyethylensorbitanfettsäureester oder Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cerylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe etc.

Salben sind Wasser-in-Oel-Emulsionen, die bis zu 70%, vorzugsweise jedoch von etwa 20% bis etwa 50% Wasser oder wässrige Phasen enthalten.

Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Puder enthalten neben der Wirksubstanz z.B. obengenannte sekretabsorbierende Puderbestandteile.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens wässrig-ethanolische Grundlage auf, der u.a. Polyalkohole, z.B. Glycerin, Glykole, und/oder Poly-Ethylenglykol, als Feuchthaltemittel zur Herabsetzung der Verdunstung, und rückfettende Substanzen, wie Fettsäureester mit niedrigen Poly-Ethylenglykolen, d.h. im wässrigen Gemisch lösliche, lipophile Substanzen als Ersatz für die der Haut mit dem Ethanol entzogene Fettsubstanzen, und, falls notwendig, andere Hilfs- und Zusatzmittel zugegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

In den nachfolgenden Beispielen sind Temperaturen in Celsiusgraden angegeben, Prozentangaben beziehen sich stets auf Gewicht.

Sofern nicht besonders vermerkt, ist bei der Nennung eines Wirkstoffes der Formel I stets das racemische bzw. Diastereomerengemisch gemeint. Erfolgt keine gezielte Synthese reiner Isomeren, so kann ein Produkt der Formel I in Form eines Gemisches der möglichen Isomeren anfallen.

Herstellungsbeispiele:

Beispiel 1: Herstellung von

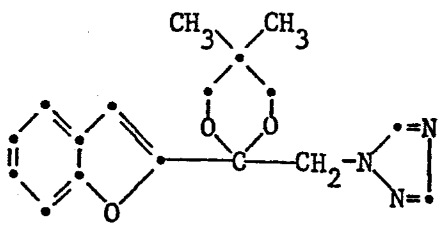

(1.18)

2-(Benzofuran-2-yl)-2-[1-(1H-1,2,4-triazolyl)-methyl]-5,5-dimethyl-1,3-dioxan

a) Herstellung des Zwischenproduktes

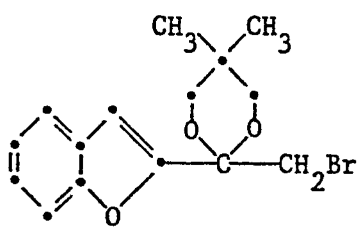

2-(Benzofuran-2-yl)-2-brommethyl-5,5-dimethyl-1,3-dioxan

48 g 2-Acetylbenzofuran und 55 g 2,2-Dimethyl-1,3-propandiol in 400 ml 1,1,1-Trichlorethan werden in Gegenwart von 2 g katalytisch wirkender p-Toluolsulfonsäure 30 Stunden unter Rückfluss erhitzt, wobei gebildetes Wasser mittels eines Wasserabscheiders abgetrennt wird. Anschliessend lässt man 49,6 g Brom in 50 ml 1,1,1-Trichlorethan innerhalb von 45 Minuten zutropfen und erhitzt das Reaktionsgemisch weitere 2 Stunden unter Rückfluss. Nach dem Abkühlen auf Raumtemperatur wird das Reaktionsgemisch dreimal mit je 100 ml Wasser gewaschen, über Natriumsulfat getrocknet, filtriert, das Lösungsmittel verdampft und das ölige Rohprodukt durch fraktionierte Destillation im Hochvakuum gereinigt. Sdp. 130-140°/0,04 mbar.

b) Herstellung des Endproduktes:

18,4 g 1,2,4-Triazol, 11,7 g Kaliumcarbonat und eine katalytisch wirkende Menge Natriumjodid in 100 ml Dimethylsulfoxid werden zusammen mit 18,4 g des unter a) hergestellten 2-(Benzofuran-2-yl)-2-brommethyl-5,5-dimethyl-1,3-dioxans 30 Stunden bei einer Innentemperatur von +120° gerührt. Nach dem Abkühlen auf Raumtemperatur werden 600 ml Wasser zugegeben, dreimal mit je 200 ml Ethylacetat extrahiert, die vereinigten Extrakte zweimal mit je 100 ml Wasser gewaschen, über Natriumsulfat getrocknet, filtriert und das Lösungsmittel verdampft. Der ölige Rückstand wird über Kieselgel 60 mit Ethylacetat als Laufmittel säulenchromatographisch gereinigt. Nach Verdampfen des Laufmittels wird der ölige Rückstand durch Zugabe von Diethylether zur Kristallisation gebracht. Die farblosen Kristalle schmelzen bei 102,5-106°.

Beispiel 2: Herstellung von

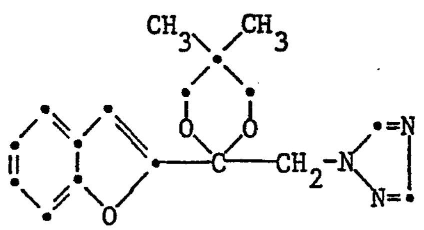

• $HNO_3$ (1.19)

Nitrat von 2-(Benzofuran-2-yl)-2-[1-(1H-1,2,4-triazolyl)-methyl]-5,5-dimethyl-1,3-dioxan

5 g 2-(Benzofuran-2-yl)-2-[1-(1H-1,2,4-triazolyl)-methyl]-5,5-dimethyl-1,3-dioxan in 100 ml Diisopropylether und 20 ml Dioxan werden mit 5 ml 65%iger Salpetersäure versetzt. Nach dem Abklingen der schwach exothermen Reaktion fällt das Salz als farbloses, kristallines Pulver aus; es wird abfiltriert und mit Diisopropylether gewaschen. Nach Entfernen des Lösungsmittels im Vakuum schmilzt das Nitrat bei 169-171° unter Zersetzung.

Beispiel 3: Herstellung von

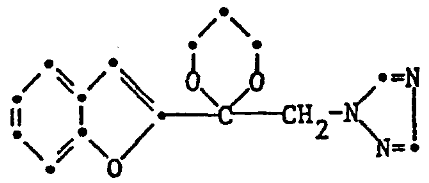

• HCl (1.36) ,

Hydrochlorid von 2-(Benzofuran-2-yl)-2-[1-(1H-1,2,4-triazolyl)-methyl]-1,3-dioxan

3,13 g 2-(Benzofuran-2-yl)-2-[1-(1H-1,2,4-triazolyl)-methyl]-1,3-dioxan werden in 50 ml Ethylacetat gelöst und mit 100 ml Diethylether versetzt. Beim Zutropfen von 1,1 g konz. Salzsäure unter Rühren bildet sich eine beige Kristallsuspension, die nach 20 Minuten abfiltriert und mit 100 ml Diethylether gewaschen wird. Nach dem Trocknen im Vakuum schmilzt das Hydrochlorid bei 192-197°.

Beispiel 4: Herstellung von

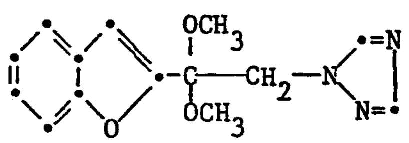

(1.37)

1-(Benzofuran-2-yl)-1,1-dimethoxy-2-[1-(1H-1,2,4-triazolyl)]-ethan

a) Herstellung des Zwischenproduktes

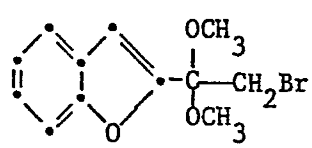

1-(Benzofuran-2-yl)-1,1-dimethoxy-2-brom-ethan

33,2 g 2-(2-Bromacetyl)-benzofuran
29,5 g Orthoameisensäuretrimethylester und
2,6 g p-Toluolsulfonsäure in 275 ml Methanol werden 15 Stunden zum Rückfluss erhitzt. Anschliessend wird das Methanol im Vakuum abgezogen und der Rückstand in 250 ml Diethylether gelöst. Die etherische Lösung wird einmal mit 125 ml n-Natronlauge und zweimal mit je 125 ml Wasser

gewaschen, über Natriumsulfat getrocknet, filtriert und das Lösungsmittel im Vakuum entfernt. Der ölige Rückstand wird durch Zugabe von Hexan zur Kristallisation gebracht. Nach Umkristallisieren aus Ethanol schmelzen die gelben Kristalle bei 72-73°.

b) Herstellung des Endproduktes:

9 g 1,2,4-Triazol, 18 g Kaliumcarbonat, 1 g Kaliumjodid und 28,5 g des unter a) hergestellten 1-(Benzofuran-2-yl)-1,1-dimethoxy-2-bromethan in 150 ml Dimethylsulfoxid werden bei einer Innentemperatur von +120° 15 Stunden gerührt. Nach Zugabe von weiteren 3,5 g 1,2,4-Triazol und 1 g Kaliumjodid wird bei einer Innentemperatur von +140° noch 20 Stunden weitergerührt. Anschliessend wird das braune Reaktionsgemisch auf Raumtemperatur abgekühlt, auf 600 ml Eiswasser gegossen und dreimal mit je 200 ml Ethylacetat extrahiert. Die vereinigten Extrakte werden zweimal mit je 200 ml Wasser gewaschen, über Natriumsulfat getrocknet, filtriert und das Lösungsmittel im Vakuum entfernt. Der ölige Rückstand wird durch Zugabe von Petrolether zur Kristallisation gebracht. Nach dem Umkristallisieren aus Isopropanol schmilzt die Verbindung Nr. 1.37 bei 120-123°.

Beispiel 5: Herstellung von

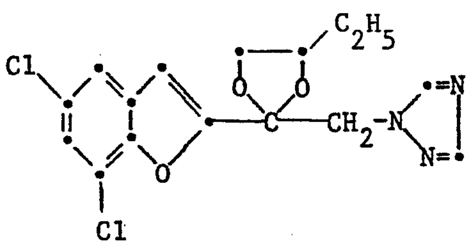

(1.5)

2-(5,7-Dichlor-benzofuran-2-yl)-2-[1-(1H-1,2,4-triazolyl)-methyl]-4-ethyl-1,3-dioxolan

10,8 g des Nitrats vom 1-(Benzofuran-2-yl)-2-(1H-1,2,4-triazolyl-1-yl)-ethanon, 7 g 1,2-Butandiol, 4,9 g p-Toluolsulfonsäure, 20 g 1-Pentanol und 200 ml Xylol werden 3 Tage unter Rückfluss

erhitzt, wobei gebildetes Wasser mittels eines Wasserabscheiders abgetrennt wird. Nach dem Abkühlen auf Raumtemperatur wird zweimal mit je 200 ml verdünnter Natronlauge.zweimal mit je 100 ml Wasser gewaschen, über Natriumsulfat getrocknet, filtriert und das Lösungsmittel verdampft. Der ölige Rückstand wird über Kieselgel mit Ethylacetat als Laufmittel säulenchromatographisch gereinigt. Nach dem Verdampfen des Laufmittels kristallisiert der ölige Rückstand langsam aus. Die beigen Kristallen schmelzen bei 102,5-105°.

Beispiel 6: Herstellung von

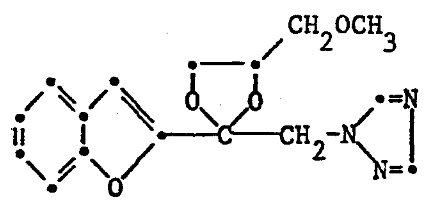

(1.11)

2-(Benzofuran-2-yl)-2-[1-(1H-1,2,4-triazolyl)-methyl]-4-methoxymethyl-1,3-dioxolan

12,5 g 2-(Benzofuran-2-yl)-2-[1-(1H-1,2,4-triazolyl)-methyl]-4-hydroxymethyl-1,3-dioxolan werden in 150 ml N,N-Dimethylformamid gelöst, unter Durchleiten von Stickstoff und Rühren mit 1,9 g 55 %iger Natriumhydrid-Dispersion versetzt und 2 Stunden auf 80° erwärmt. Nach dem Abkühlen auf Raumtemperatur werden unter Rühren innerhalb 1 Stunde 6,3 g Methyljodid zugetropft. Das Reaktionsgemisch wird 2 Stunden auf 60° erwärmt, auf Raumtemperatur abgekühlt, mit 800 ml Eiswasser verdünnt und dreimal mit je 300 ml Ethylacetat extrahiert. Die vereinigten Extrakte werden zweimal mit je 30 ml Wasser gewaschen, über Natriumsulfat getrocknet, filtriert. Das Lösungsmittel wird verdampft und der Rückstand an Kieselgel mit Aceton/Ethylacetat (1:1) als Laufmittel säulenchromatographisch gereinigt. Nach dem Verdampfen des Laufmittelgemisches verbleibt ein zähflüssiges Oel; $n_D^{25} = 1,5540$.

Beispiel 7: Auf analoge Weise wie in Beispiel 1 bis 6 beschrieben lassen sich folgende Verbindungen der Formel I (falls nicht besonders vermerkt, als Diastereomerengemische mit unterschiedlichen Mischungsverhältnissen) herstellen:

Tabelle 1: Verbindungen der Formel I mit Y = -N=

| Verb. Nr. | $R_1$ | $R_2$ | A | B | Salz bzw. Komplex | Physik. Konst. [°C] |
|---|---|---|---|---|---|---|
| 1.1 | H | H | $-CH_2-CH(CH_3)-$ | | - | $n_D^{54}$ = 1.5540 |
| 1.2 | H | H | $-CH_2-CH_2-$ | | $HNO_3$ | |
| 1.3 | H | H | $-CH_2-CH_2-$ | | - | Smp. 96-99 |
| 1.4 | H | H | $-CH_2-CH_2-$ | | $Mn(NO_3)_2$ | |
| 1.5 | 5-Cl | 7-Cl | $-CH_2-CH(C_2H_5)-$ | | - | Smp. 102,5-105 |
| 1.6 | 5-Cl | 7-Cl | $-CH_2-CH_2-$ | | - | Smp. 154-156 |
| 1.7 | $5-CH_3$ | $7-CH_3$ | $-CH_2-CH_2-$ | | - | |
| 1.8 | 5-Cl | 7-Cl | $-CH_2-CH(CH_3)-$ | | - | Smp. 117-118 |
| 1.9 | $5-OCH_3$ | $7-OCH_3$ | $-CH_2-CH_2-$ | | - | $n_D^{26}$ = 1,5638 |
| 1.10 | 5-Cl | 7-Cl | $-CH_2-CH(CH_2OCH_3)-$ | | - | $n_D^{26}$ = 1.5712 |
| 1.11 | H | H | $-CH_2-CH(CH_2OCH_3)-$ | | - | $n_D^{25}$ = 1.5540 |
| 1.12 | H | H | $-CH_2-CH(CH_2OCH_3)-$ | | $CuCl_2$ | |
| 1.13 | H | H | $-CH_2CH(CH_2OC_2H_5)-$ | | - | $n_D^{25}$ = 1.5476 |
| 1.14 | H | H | $-CH_2-CH(CH_2OC_6H_5)-$ | | - | Smp. 131-135 |
| 1.15 | 5-Cl | 7-Cl | $-CH_2-CH(CH_2OC_2H_5)-$ | | - | $n_D^{26}$ = 1.5632 |
| 1.16 | $5-OC_2H_5$ | $7-OC_2H_5$ | $-CH(CH_3)-CH(CH_3)-$ | | HCl | |
| 1.17 | H | H | $-CH(CH_3)-CH(CH_3)-$ | | - | Smp. 99-102 |

Tabelle 1: Verbindungen der Formel I mit Y = -N= (Fortsetzung)

| Verb. Nr. | $R_1$ | $R_2$ | A B | Salz bzw. Komplex | Physik. Konst. [°C] |
|---|---|---|---|---|---|
| 1.18 | H | H | $-CH_2-C(CH_3)_2-CH_2-$ | - | Smp. 102,5-106 |
| 1.19 | H | H | $-CH_2-C(CH_3)_2-CH_2-$ | $HNO_3$ | Smp. 169-171/Zers. |
| 1.20 | H | H | $-CH_2-C(CH_3)_2-CH_2-$/trans | - | |
| 1.21 | H | H | $-CH_2-C(CH_3)_2-CH_2-$/cis | - | |
| 1.22 | H | H | $-CH_2-C(CH_3)_2-CH_2-$ | $CuSO_4 \cdot H_2O$ | Smp. 238-241 |
| 1.23 | 5-Cl | 7-Cl | $-CH_2-C(CH_3)_2-CH_2-$ | - | Smp. 149-152 |
| 1.24 | H | H | $-CH_2-C(CH_3)(C_2H_5)-CH_2-$ | - | Smp. 89-99 |
| 1.25 | H | H | $-CH_2-C(CH_3)(C_3H_7-n)-CH_2$ | - | Smp. 142,5-145 |
| 1.26 | 5-$CH_3$ | 7-$CH_3$ | $-CH_2-C(CH_3)_2-CH_2-$ | - | |
| 1.27 | H | H | $-CH_2-C(CH_3)_2-CH(C_3H_7-n)-$ | - | $n_D^{26}$ = 1.5430 |
| 1.28 | H | H | $-CH_2-C(C_2H_5)_2-CH_2-$ | - | Smp. 114-116 |
| 1.29 | H | H | $-CH(CH_3)-CH_2-C(CH_3)_2-$ | - | Smp. 130-136 |
| 1.30 | 5-$OCH_3$ | 7-$OCH_3$ | $-CH(CH_3)-CH_2-C(CH_3)_2-$ | $ZnCl_2$ | |
| 1.31 | H | H | $-CH_2-CH_2-CH(CH_3)-$ | | Smp. 94-100 |
| 1.32 | H | H | $-CH_2-C(C_2H_5)(C_4H_9-n)-CH_2-$ | - | Smp. 127-132 |
| 1.33 | H | H | $-CH_2-CH(C_2H_5)-CH(C_3H_7-n)-$ | - | $n_D^{26}$ = 1.5428 |
| 1.34 | H | H | $-CH_2-CH_2-CH_2-$ | $CF_3COOH$ | |

Tabelle 1: Verbindungen der Formel I mit Y = -N= (Fortsetzung)

| Verb. Nr. | $R_1$ | $R_2$ | A | B | Salz bzw. Komplex | Physik. Konst. [°C] |
|---|---|---|---|---|---|---|
| 1.35 | H | H | $-CH_2-CH_2-CH_2-$ | | $Mn(NO_3)_2$ | |
| 1.36 | H | H | $-CH_2-CH_2-CH_2-$ | | - | Smp. 93-94,5 |
| 1.37 | H | H | $CH_3$ | $CH_3$ | - | Smp. 120-123 |
| 1.38 | H | H | $CH_3$ | $CH_3$ | HCl | |
| 1.39 | 5-Cl | 7-Cl | $CH_3$ | $CH_3$ | - | |
| 1.40 | 5-$CH_3$ | 7-$CH_3$ | $CH_3$ | $CH_3$ | - | |
| 1.41 | 5-$CH_3$ | 7-$CH_3$ | $CH_3$ | $CH_3$ | $ZnCl_2$ | |
| 1.42 | H | H | $C_2H_5$ | $C_2H_5$ | - | |
| 1.43 | H | H | $C_2H_5$ | $C_2H_5$ | $Mn(NO_3)_2$ | |
| 1.44 | H | H | $C_3H_7$-n | $C_3H_7$-n | - | |
| 1.45 | 5-Cl | 7-Cl | $C_3H_7$-n | $C_3H_7$-n | - | |
| 1.46 | 5-$OCH_3$ | 7-$OCH_3$ | $CH_3$ | $CH_3$ | - | |
| 1.47 | H | H | $-CH_2-CH(CH_2OH)-$ | | - | Smp. 121-126 |
| 1.48 | H | H | $-CH_2-CH_2-CH_2-$ | | HCl | Smp. 192-197 |
| 1.49 | H | H | $-CH_2-CH(C_2H_5)-$ | | - | $n_D^{25}$ = 1,5703 |
| 1.50 | 5-Cl | 7-Cl | $-CH_2-CH(C_3H_7-n)-$ | | - | |

Tabelle 1: Verbindungen der Formel I mit Y = -N= (Fortsetzung)

| Verb. Nr. | $R_1$ | $R_2$ | A B | Salz bzw. Komplex | Physik. Konst. [°C] |
|---|---|---|---|---|---|
| 1.51 | H | H | $-CH(CH_3)-CH(C_2H_5)-$ | - | |
| 1.52 | 5-Cl | 7-Cl | $-CH_2CH(CH_2OH)-$ | - | |
| 1.53 | 5-Cl | 7-Cl | $-CH_2-CH_2-CH_2-$ | - | Smp. 98-103 |
| 1.54 | H | H | $-CH_2-CH(CH_2OC_3H_7-n)-$ | - | |
| 1.55 | 5-Cl | 7-Cl | $-CH_2-CH_2-CH(CH_3)-$ | - | |
| 1.56 | H | H | $-CH_2-CH(C_3H_7-n)-$ | - | |
| 1.57 | 5-Cl | 7-Cl | $-CH(CH_3)-CH(CH_3)-$ | - | Smp. 111-116 |
| 1.58 | 5-Cl | 7-Cl | $-CH(CH_3)-CH(C_2H_5)-$ | - | |
| 1.59 | 5-Cl | 7-Cl | $-CH_2-CH(CH_2OC_3H_7-n)-$ | - | Smp. 89-94 |
| 1.60 | 5-Cl | 7-Cl | $-CH_2-CH(CH_2OC_3H_7-i)-$ | - | $n_D^{26}$ = 1,5428 |
| 1.61 | H | H | $-CH_2-CH(CH_2OC_3H_7-i)-$ | - | |
| 1.62 | 5-Cl | H | $-CH_2-CH(CH_3)-$ | - | $n_D^{25}$ = 1,5493 |
| 1.63 | $5-OCH_3$ | H | $-CH_2-CH_2-$ | - | |
| 1.64 | $5-OCH_3$ | H | $-CH_2-CH(CH_3)-$ | - | |

**Tabelle 1**: Verbindungen der Formel I mit Y = -N= (Fortsetzung)

| Verb. Nr. | $R_1$ | $R_2$ | A – B | Salz bzw. Komplex | Physik. Konst. [°C] |
|---|---|---|---|---|---|
| 1.65 | 5-Cl | H | $-CH_2-CH_2-$ | – | Smp. 75-81 |
| 1.66 | $5-CH_3$ | $7-CH_3$ | $-CH_2-CH(CH_2OCH_3)-$ | – | |
| 1.67 | $5-OCH_3$ | H | $-CH_2-CH(CH_2OCH_3)-$ | – | |
| 1.68 | $5-CH_3$ | $7-CH_3$ | $-CH_2-CH_2-CH_2-$ | – | |
| 1.69 | 5-Cl | H | $-CH_2-CH(C_2H_5)-$ | – | Smp. 92-97 |
| 1.70 | $5-OCH_3$ | H | $-CH_2-CH(C_2H_5)-$ | – | |
| 1.71 | $5-CH_3$ | $7-OCH_3$ | $-CH_2-CH_2-$ | – | |
| 1.72 | 5-Cl | H | $-CH(CH_3)-CH(CH_3)-$ | – | |
| 1.73 | $5-OCH_3$ | H | $-CH_2-CH(CH_2OC_2H_5)-$ | – | |
| 1.74 | $5-CH_3$ | $7-OCH_3$ | $-CH_2-CH(CH_3)-$ | – | |
| 1.75 | $5-CH_3$ | $7-OCH_3$ | $-CH_2-CH(CH_2OCH_3)-$ | – | |
| 1.76 | 5-Cl | H | $-CH_2-CH(CH_2OCH_3)-$ | – | |
| 1.77 | $5-CH_3$ | $7-OCH_3$ | $-CH_2-CH_2-CH_2-$ | – | |

Tabelle 1: Verbindungen der Formel I mit Y = -N= (Fortsetzung)

| Verb. Nr. | $R_1$ | $R_2$ | A | B | Salz bzw. Komplex | Physik. Konst. [°C] |
|---|---|---|---|---|---|---|
| 1.78 | $5-OCH_3$ | H | $-CH(CH_3)-CH(CH_3)-$ | | - | |
| 1.79 | 5-Cl | H | $-CH_2-CH(CH_2OC_2H_5)-$ | | - | |
| 1.80 | $5-CH_3$ | H | $-CH_2-CH(C_2H_5)-$ | | - | |
| 1.81 | $5-CH_3$ | $7-CH_3$ | $-CH_2-CH(CH_3)-$ | | - | |
| 1.82 | 5-Cl | H | $-CH_2-CH_2-CH_2-$ | | - | $n_D^{26} = 1,5391$ |
| 1.83 | $5-OCH_3$ | H | $-CH_2-CH_2-CH_2-$ | | - | |
| 1.84 | $5-CH_3$ | $7-CH_3$ | $-CH_2-CH(C_2H_5)_2-$ | | - | |
| 1.85 | $5-CH_3$ | H | $-CH_2-CH(CH_2OCH_3)-$ | | - | |
| 1.86 | $5-CH_3$ | H | $-CH_2-CH_2-$ | | - | |
| 1.87 | $5-CH_3$ | $7-OCH_3$ | $-CH_2-CH(CH_2OC_2H_5)-$ | | - | |
| 1.88 | $5-CH_3$ | H | $-CH_2-CH(CH_3)-$ | | - | |

Tabelle 2: Verbindungen der Formel I mit Y = $-CH=$

| Verb. Nr. | $R_1$ | $R_2$ | A | B | Salz bzw. Komplex | Physik. Konst. [°C] |
|---|---|---|---|---|---|---|
| 2.1 | H | H | $-CH_2-CH_2-$ | | - | Smp. 81-83 |
| 2.2 | $5-CH_3$ | $7-CH_3$ | $-CH_2-CH_2-$ | | $ZnCl_2$ | |
| 2.3 | 5-Cl | 7-Cl | $-CH_2-CH(C_2H_5)-$ | | - | dunkles Oel |
| 2.4 | 5-Cl | 7-Cl | $-CH_2-CH(CH_3)-$ | | - | $n_D^{54} = 1.5755$ |
| 2.5 | $5-OCH_3$ | $7-OCH_3$ | $-CH(CH_3)-CH(CH_3)-$ | | $HNO_3$ | |
| 2.6 | 5-Cl | 7-Cl | $-CH_2-CH(CH_2OC_2H_5)-$ | | - | $n_D^{26} = 1.5672$ |
| 2.7 | H | H | $-CH_2-CH(CH_2OCH_3)-$ | | - | $n_D^{25} = 1.5570$ |
| 2.8 | H | H | $-CH_2-CH_2-CH_2-$ | | - | $n_D^{26} = 1.5712$ |
| 2.9 | H | H | $-CH_2-CH_2-CH_2-$ | | $Mn(NO_3)_2$ | |
| 2.10 | 5-Cl | 7-Cl | $-CH_2-CH(CH_2OCH_3)-$ | | - | $n_D^{26} = 1.5755$ |
| 2.11 | 5-Cl | 7-Cl | $-CH_2-CH_2-$ | | - | $n_D^{54} = 1.5732$ |
| 2.12 | H | H | $-CH(CH_3)-CH_2-C(CH_3)_2-$ | | - | |
| 2.13 | H | H | $-CH(CH_3)-CH_2-C(CH_3)_2-$ | | $CuCl_2$ | |
| 2.14 | H | H | $-CH_2-CH_2-CH(CH_3)-$ | | - | |
| 2.15 | H | H | $-CH_2-CH_2-CH(CH_3)-$ | | $CCl_3COOH$ | |
| 2.16 | H | H | $-CH_2-C(CH_3)_2-CH_2-$ | | - | Smp. 104-106 |

Tabelle 2: Verbindungen der Formel I mit Y = -CH= (Fortsetzung)

| Verb. Nr. | $R_1$ | $R_2$ | A | B | Salz bzw. Komplex | Physik. Konst. [°C] |
|---|---|---|---|---|---|---|
| 2.17 | 5-Cl | 7-Cl | $-CH_2-C(CH_3)_2-CH_2-$ | | HCl | |
| 2.18 | H | H | $-CH_2-C(CH_3)_2-CH_2-$ | | $ZnCl_2$ | |
| 2.19 | H | H | $CH_3$ | $CH_3$ | - | |
| 2.20 | H | H | $CH_3$ | $CH_3$ | $HNO_3$ | |
| 2.21 | H | H | $CH_3$ | $CH_3$ | $CuSO_4$ | |
| 2.22 | 5-Cl | 7-Cl | $CH_3$ | $CH_3$ | - | |
| 2.23 | $5-CH_3$ | $7-CH_3$ | $CH_3$ | $CH_3$ | - | |
| 2.24 | H | H | $C_2H_5$ | $C_2H_5$ | - | |
| 2.25 | H | H | $C_3H_7$-n | $C_3H_7$-n | $ZnCl_2$ | |
| 2.26 | H | H | $C_3H_7$-n | $C_3H_7$-n | - | |
| 2.27 | H | H | $-CH-CH(CH_2OC_2H_5)-$ | | - | |
| 2.28 | 5-Cl | 7-Cl | $-CH_2-CH(C_3H_7-n)-$ | | - | |
| 2.29 | H | H | $-CH_2-CH(CH_3)-$ | | - | |
| 2.30 | 5-Cl | 7-Cl | $-CH_2-CH_2-CH_2-$ | | - | $n_D^{26} = 1.5697$ |

Tabelle 2: Verbindungen der Formel I mit Y = -CH= (Fortsetzung)

| Verb. Nr. | $R_1$ | $R_2$ | A B | Salz bzw. Komplex | Physik. Konst. [°C] |
|---|---|---|---|---|---|
| 2.31 | 5-Cl | 7-Cl | $-CH(CH_3)-CH(CH_3)-$ | - | Smp. 75-81 |
| 2.32 | H | H | $-CH_2-CH(C_3H_7\text{-}n)-$ | - | |
| 2.33 | H | H | $-CH_2-CH(CH_2OC_3H_7\text{-}n)-$ | - | |
| 2.34 | 5-Cl | H | $-CH_2-CH_2-$ | - | $n_D^{25}$= 1.5598 |
| 2.35 | H | H | $-CH_2-CH(C_2H_5)-$ | - | |
| 2.36 | $5\text{-}CH_3$ | H | $-CH_2-CH_2-$ | - | $n_D^{25}$= 1.5632 |
| 2.37 | 5-Cl | H | $-CH_2-CH_2-CH_2-$ | - | |
| 2.38 | H | H | $-CH(CH_3)-CH(CH_3)-$ | - | |
| 2.39 | $5\text{-}CH_3$ | H | $-CH_2-CH(CH_3)-$ | - | |
| 2.40 | 5-Cl | 7-Cl | $-CH_2-CH(CH_2OC_3H_7\text{-}n)-$ | - | |
| 2.41 | $5\text{-}OCH_3$ | H | $-CH_2-CH_2-$ | - | |
| 2.42 | 5-Cl | H | $-CH_2-CH(CH_3)-$ | - | |

Tabelle 2: Verbindungen der Formel I mit Y = -CH= (Fortsetzung)

| Verb. Nr. | $R_1$ | $R_2$ | A | B | Salz bzw. Komplex | Physik. Konst. [°C] |
|---|---|---|---|---|---|---|
| 2.43 | 5-Cl | 7-Cl | $-CH_2-CH_2-CH(CH_3)-$ | | - | |
| 2.44 | $5-CH_3$ | H | $-CH_2-CH(CH_2OCH_3)-$ | | - | |
| 2.45 | $5-OCH_3$ | H | $-CH_2-CH(CH_3)-$ | | - | |
| 2.46 | $5-OCH_3$ | $7-OCH_3$ | $-CH_2-CH(CH_2OCH_3)-$ | | - | |
| 2.47 | $5-OCH_3$ | $7-OCH_3$ | $-CH_2-CH(CH_3)-$ | | - | |
| 2.48 | 5-Cl | H | $-CH(CH_3)-CH(CH_3)-$ | | - | |
| 2.49 | $5-OCH_3$ | $7-OCH_3$ | $-CH_2-CH_2-CH_2-$ | | - | |
| 2.50 | $5-OCH_3$ | $7-OCH_3$ | $-CH_2-CH(C_2H_5)-$ | | - | |
| 2.51 | 5-Cl | H | $-CH_2-CH(CH_2OCH_3)-$ | | - | |

Beispiel 8:

Formulierungsbeispiele für pharmazeutische Präparate:

A. Salben:

Eine Salbe, enthaltend 5% 2-(Benzofuran-2-yl)-2-[1-(1H-1,2,4-triazolyl)-methyl]-5,5-dimethyl-1,3-dioxan, kann wie folgt hergestellt werden:

Zusammensetzung:

| | |
|---|---|
| Wirkstoff | 5,0% |
| Vaseline | 45,0% |
| Paraffinöl | 19,6% |
| Cetylalkohol | 5,0% |
| Bienenwachs | 5,0% |
| Sorbitan-sesquioleat | 5,0% |
| p-Hydroxybenzoesäureester | 0,2% |
| Wasser, entmineralisiert bis zu | 100,0% |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

B. Creme

Creme, enthaltend 10% 2-(Benzofuran-2-yl)-2-[1-(1H-1,2,4-triazolyl)-methyl]-5,5-dimethyl-1,3-dioxan, kann wie folgt hergestellt werden:

Zusammensetzung:

| | |
|---|---|
| Wirkstoff | 10,0% |
| Isopropylpalmitat | 8,0% |
| Cetylpalmitat | 1,5% |
| Siliconöl 100 | 0,5% |
| Sorbitanmonostearat | 3,0% |
| Polysorbat 60 | 3,5% |
| 1,2-Propylenglykol PH | 20,0% |
| Acrylsäurepolymerisat | 0,5% |
| Triethanolamin | 0,7% |
| Wasser, entmineralisiert bis zu | 100,0% |

Das Acrylsäurepolymerisat wird in einem Gemisch aus entmineralisiertem Wasser und 1,2-Propylenglykol suspendiert. Unter Rühren wird hierauf Triethanolamin zugegeben, wodurch ein Schleim erhalten wird. Ein Gemisch aus Isopropylpalmitat, Cetylpalmitat, Siliconöl, Sorbitanmonostearat und Polysorbat wird auf ca. 75° erwärmt und unter Rühren in den gleichfalls auf ca. 75° erwärmten Schleim eingearbeitet. Die auf Raumtemperatur abgekühlte Creme-Grundlage wird hierauf zur Herstellung eines Konzentrates mit dem Wirkstoff verwendet. Das Konzentrat wird mittels eines Durchlaufhomogenisators homogenisiert und dann portionenweise dem Rest der Grundlage hinzugefügt.

Creme, enthaltend 5% 2-(2-Benzofuran-2-yl)-2-[1-(1H-1,2,4-triazolyl)-methyl]-5,5-dimethyl-1,3-dioxan, kann wie folgt hergestellt werden:

Zusammensetzung:

| | |
|---|---|
| Wirkstoff | 5,0% |
| Cetylpalmitat PH | 2,0% |
| Cetylalkohol PH | 2,0% |
| Triglyceridgemisch gesättigter mittelfettiger Fettsäuren | 5,0% |
| Stearinsäure | 3,0% |
| Glycerinstearat PH | 4,0% |
| Cetomacrogol 1000 | 1,0% |
| mikrokristalline Cellulose | 0,5% |
| 1,2-Propylenglykol, dest. | 20,0% |
| Wasser, entmineralisiert bis zu | 100,0% |

Cetylalkohol, Cetylpalmitat, das Triglyceridgemisch, Stearinsäure und Glycerinstearat werden zusammengeschmolzen. Die mikrokristalline Cellulose wird in einen Teil des Wassers dispergiert. Im restlichen Teil des Wasser wird Cetomacrogol gelöst und das Propylenglykol sowie der Schleim dazu gemischt. Die Fettphase wird anschliessend unter Rühren zur Wasserphase gegeben und kaltgerührt. Schliesslich wird der Wirkstoff mit einem Teil der Grundlage angerieben und hierauf die Anreibung in den Rest der Creme eingearbeitet.

C. Hydrogele:

Ein transparentes Hydrogel, enthaltend 5% 2-(2-Benzofuran-2-yl)-2-[1-(1H-1,2,4-triazolyl)-methyl]-5,5-dimethyl-1,3-dioxan, wird wie folgt hergestellt:

Zusammensetzung:

| | |
|---|---|
| Wirkstoff | 5% |
| Propylenglykol | 10-20% |
| Isopropanol | 20% |
| Hydroxypropyl-methylcellulose | 2% |
| Wasser | ad 100% |

Die Hydroxypropyl-methylcellulose wird im Wasser gequollen. Der Wirkstoff wird in einem Gemisch aus Isopropanol und Propylenglykol gelöst. Anschliessend wird die Wirkstofflösung mit einem gequollenen Cellulose-Derivat vermischt und, wenn erwünscht, mit Riechstoffen (0,1%) versetzt.

D. Schaumsprays

Ein Schaumspray, enthaltend 1% 2-(Benzofuran-2-yl)-2-[1-(1H-1,2,4-triazolyl)-methyl]-5,5-dimethyl-1,3-dioxan, kann folgendermassen hergestellt werden:

Zusammensetzung:

| | |
|---|---|
| Wirkstoff | 1,00% |
| Cerylalkohol PH | 1,70% |
| Paraffinöl, dickflüssig | 1,00% |
| Isopropylmyristat | 2,00% |
| Cetamacrogol | 2,40% |
| Sorbitanmonostearat | 1,50% |
| 1,2-Propylenglykol PH | 5,00% |
| Methylparaben | 0,18% |
| Propylpareben | 0,02% |
| Chemoderm 314 | 0,10% |
| Wasser, entmineralisiert bis zu | 100,00% |

Cetylalkohol, Paraffinöl, Isopropylmyristat, Cetomacrogol und Sorbitanstearat werden zusammengeschmolzen. Methyl- und Propylparaben werden in heissem Wasser gelöst. Die Schmelze und die Lösung werden anschliessend vermischt. Der Wirkstoff, in Propylenglykol suspendiert, wird in die Grundlage eingearbeitet. Anschliessend wird Chemoderm zugeführt und mit Wasser auf das Endgewicht ergänzt.

Abfüllung:

20 ml des Gemisches werden in eine Aluminiumblockdose eingefüllt. Die Dose wird mit einem Ventil versehen, und das Treibgas wird unter Druck eingefüllt.

E. Kapseln

Gelatinekapseln, enthaltend 200 mg 2-(Benzofuran-2-yl)-2-[1-(1H-1,2,4-triazolyl)-methyl]-5,5-dimethyl-1,3-dioxan als Wirkstoff, können z.B. wie folgt hergestellt werden:

| Zusammensetzung (für 1000 Kapseln) | |
|---|---|
| Wirkstoff | 100 g |
| Laktose, gemahlen | 100 g |

Der Wirkstoff und die Laktose (feinst gemahlen) werden gut miteinander vermischt. Das erhaltene Pulver wird gesiebt und in Portionen zu je 0,20 g in Gelatinekapseln abgefüllt.

F. Tabletten

Tabletten, enthaltend 25 mg Wirkstoff, z.B. 2-(Benzofuran-2-yl)-2-[1-(1H-1,2,4-triazolyl)-methyl]-5,5-dimethyl-1,3-dioxan, können folgendermassen erhalten werden:

| Bestandteile (für 1000 Tabletten) | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Tabletten enthaltend 100 mg Wirkstoff, z.B. 2-(Benzofuran-2-yl)-2-[1-(1H-1,2,4-triazolyl)-methyl]-5,5-dimethyl-1,3-dioxan, können folgendermassen hergestellt werden:

| Bestandteile (für 1000 Tabletten) | |
|---|---|
| Wirkstoff | 100,0 g |
| Lactose | 50,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.w. |

Herstellung: Sämtliche festen Ingredienzien werden zunächst durch ein Sieb von 0,6 mm Maschenweite getrieben. Dann wird der Wirkstoff, die Lactode, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Tabletten enthaltend 75 mg Wirkstoff, z.B. 2-(Benzofuran-2-yl)-2-[1-(1H-1,2,4-triazolyl)-methyl]-5,5-dimethyl-1,3-dioxan, können folgendermassen hergestellt werden:

| Bestandteile (für 1000 Tabletten) | |
|---|---|
| Wirkstoff | 75,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise kann man auch pharmazeutische Präparate, enthaltend eine andere gemäss einem der Beispiele 1 bis 7 erhältliche Verbindungen herstellen.

Patentansprüche für die Vertragsstaaten: BE, CH, LI, DE, FR, IT, LU, NL, SE, GB

1. Verbindungen der Formel I

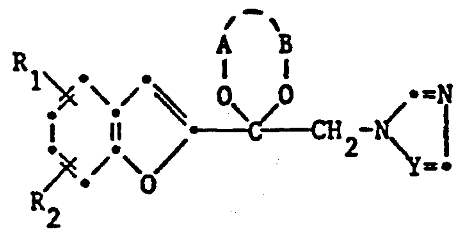

(I) ,

worin

Y für -CH= oder -N= steht:

$R_1$ und $R_2$ unabhängig voneinander für Wasserstoff, Halogen, $C_1$-$C_4$-Alkyl oder $C_1$-$C_4$-Alkoxy stehen;

A und B für $C_1$-$C_{12}$-Alkyl stehen oder zusammen eine der folgenden Alkylenbrücken

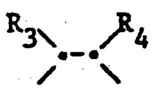 (Ia), 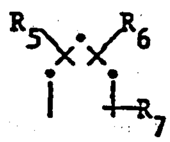 (Ib) oder 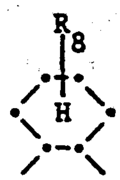 (Ic)

bedeuten, wobei

$R_3$ und $R_4$ unabhängig voneinander für Wasserstoff, ein durch Halogen oder eine Gruppe der Formel -Z-$R_9$ substituiertes oder unsubstituiertes $C_1$-$C_{12}$-Alkyl oder für ein durch Halogen oder $C_1$-$C_4$-Alkoxy und/oder $C_1$-$C_4$-Alkyl substituiertes oder unsubstituiertes Phenyl stehen, wobei Z Sauerstoff oder Schwefel bedeutet und

$R_9$ Wasserstoff, ein durch $C_1$-$C_4$-Alkoxy substituiertes oder unsubstituiertes $C_1$-$C_8$-Alkyl, $C_3$-$C_4$-Alkenyl, 2-Propinyl oder 3-Halogen-2-propinyl; ein durch Halogen, $C_1$-$C_4$-Alkyl, $C_1$-$C_4$-Alkoxy, Nitro, und/oder Trifluormethyl substituiertes oder unsubstituiertes Phenyl oder ein durch Halogen, $C_1$-$C_4$-Alkyl und/oder $C_1$-$C_4$-Alkoxy substituiertes oder unsubstituiertes Benzyl bedeutet;

$R_5$, $R_6$ und $R_7$ unabhängig voneinander Wasserstoff oder $C_1$-$C_4$-Alkyl bedeuten, wobei die Gesamtzahl der Kohlenstoffatome in $R_5$, $R_6$ und $R_7$ die Zahl 6 nicht übersteigen soll, und

$R_8$ für Wasserstoff oder $C_1$-$C_3$-Alkyl steht, sowie deren Salze.

2. Verbindungen gemäss Anspruch 1, worin in Formel I $R_1$ und $R_2$ unabhängig voneinander Wasserstoff, $C_1$-$C_4$-Alkyl, $C_1$-$C_4$-Alkoxy oder Halogen der Atomnummer bis und mit 35 bedeuten, A und B unabhängig voneinander $C_1$-$C_4$-Alkyl darstellen oder gemeinsam eine Alkylenbrücke der Formeln Ia oder Ib

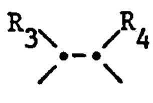 (Ia) 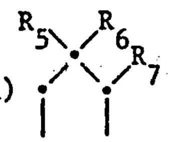 (Ib)

bilden, in der $R_3$ Wasserstoff, $C_1$-$C_4$-Alkyl oder Hydroxy-$C_1$-$C_4$-alkyl und $R_4$ Wasserstoff, $C_1$-$C_4$-Alkyl, $C_1$-$C_4$-Alkoxy-$C_1$-$C_4$-alkyl oder unsubstituiertes oder im Phenyl durch $C_1$-$C_4$-Alkyl, $C_1$-$C_4$-Alkoxy oder Halogen mit Atomnummer bis und mit 35 substituiertes Phenoxy-$C_1$-$C_4$-alkyl bedeutet bzw. $R_5$, $R_6$ und $R_7$ unabhängig voneinander Wasserstoff oder $C_1$-$C_4$-Alkyl darstellen, und Y eine Gruppe -CH= oder -N= bedeutet, und ihre Salze.

3. Verbindungen gemäss Anspruch 1 der Formel I, worin $R_1$ und $R_2$ entweder Wasserstoff oder Halogen der Atomnummer bis und mit 35, welches insbesondere in 5- und 7-Stellung gebunden ist, bedeuten, A und B unabhängig voneinander $C_1$-$C_4$-Alkyl darstellen oder gemeinsam eine Alkylenbrücke der Formeln Ia oder Ib

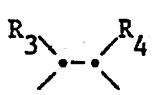 (Ia) 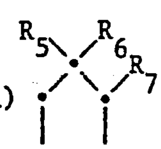 (Ib)

bilden, in der $R_3$ Wasserstoff, $C_1$-$C_4$-Alkyl oder Hydroxy-$C_1$-$C_4$-àlkyl und $R_4$ Wasserstoff, $C_1$-$C_4$-Alkyl, $C_1$-$C_4$-Alkoxy-$C_1$-$C_4$-alkyl oder unsubstituiertes oder im Phenyl durch $C_1$-$C_4$-Alkyl, $C_1$-$C_4$-Alkoxy oder Halogen mit Atomnummer bis und mit 35 substitutiertes Phenoxy-$C_1$-$C_4$-alkyl bedeutet bzw. $R_5$, $R_6$ und $R_7$ unabhängig voneinander Wasserstoff oder $C_1$-$C_4$-Alkyl darstellen, und Y eine Gruppe -CH= oder -N= bedeutet, und ihre Salze.

4. 2-(Benzofuran-2-yl)-2-[1-(1H-1,2,4-triazolyl)-methyl]-1,3-dioxolan,
2-(Benzofuran-2-yl)-2-[1-(1H-1,2,4-triazolyl)-methyl]-5-ethyl-5-methyl-1,3-dioxan,
2-(Benzofuran-2-yl)-2-[1-(1H-1,2,4-triazolyl)-methyl]-5,5-dimethyl-4-propyl-1,3-dioxan,
2-(Benzofuran-2-yl)-2-[1-(1H-1,2,4-triazolyl)-methyl]-5,5-diethyl-1,3-dioxan,
2-(Benzofuran-2-yl)-2-[1-(1H-1,2,4-triazolyl)-methyl]-4-methyl-1,3-dioxan,
2-(Benzofuran-2-yl)-2-[1-(1H-1,2,4-triazolyl)-methyl]-5-ethyl-5-butyl-1,3-dioxan,
2-(Benzofuran-2-yl)-2-[1-(1H-1,2,4-triazolyl)-methyl]-5-ethyl-4-propyl-1,3-dioxan,
2-(Benzofuran-2-yl)-2-[1-(1H-1,2,4-triazolyl)-methyl]-1,3-dioxan,
2-(Benzofuran-2-yl)-2-[1-(1H-1,2,4-triazolyl)-methyl]-4-methoxymethyl-1,3-dioxolan,
2-(Benzofuran-2-yl)-2-[1-(1H-1,2,4-triazolyl)-methyl]-4,5-dimethyl-1,3-dioxolan,
2-(Benzofuran-2-yl)-2-[1-(1H-1,2,4-triazolyl)-methyl]-4-phenoxymethyl-1,3-dioxolan,
2-(Benzofuran-2-yl)-2-(1-imidazolyl-methyl)-1,3-dioxolan,
2-(Benzofuran-2-yl)-2-(1-imidazolyl-methyl)-5,5-dimethyl-1,3-dioxan,
2-(Benzofuran-2-yl)-2-(1-imidazolyl-methyl)-4-methoxymethyl-1,3-dioxolan,
2-(5,7-Dichlor-benzofuran-2-yl)-2-[1-(1H-1,2,4-triazolyl)-methyl]-4-ethyl-1,3-dioxolan,
2-(5,7-Dichlor-benzofuran-2-yl)-2-[1-(1H-1,2,4-triazolyl)-methyl]-1,3-dioxolan,
2-(5,7-Dichlor-benzofuran-2-yl)-2-[1-(1H-1,2,4-triazolyl)-methyl]-5,5-dimethyl-1,3-dioxan,

2-(5,7-Dichlor-benzofuran-2-yl)-2-[1-(1H-1,2,4-triazolyl)-methyl]-4-ethoxymethyl-1,3-dioxolan,
2-(5,7-Dichlor-benzofuran-2-yl)-2-[1-(1H-1,2,4-triazolyl)-methyl]-4-methoxymethyl-1,3-dioxolan,
2-(5,7-Dichlor-benzofuran-2-yl)-2-[1-(1H-1,2,4-triazolyl)-methyl]-5-methyl-5-propyl-1,3-dioxan,
2-(5,7-Dichlor-benzofuran-2-yl)-2-(1-imidazolylmethyl)-1,3-dioxolan,
2-(5,7-Dichlor-benzofuran-2-yl)-2-(1-imidazolylmethyl-4-methoxymethyl-1,3-dioxolan,
2-(5,7-Dichlor-benzofuran-2-yl)-2-(1-imidazolylmethyl)-4-ethoxymethyl-1,3-dioxolan,
1-(Benzofuran-2-yl)-1,1-dimethoxy-2-[1-(1H-1,2,4-triazolyl)-ethan oder
2-(Benzofuran-2-yl)-2-[1-(1H-1,2,4-triazolyl)-methyl]-4-ethoxymethyl-1,3-dioxolan gemäss Ansprüch 1 oder jeweils ein Salz davon.

5. 2-(5,7-Dichlor-benzofuran-2-yl)-2-[1-(1H-1,2,4-triazolyl)-methyl]-4-methyl-1,3-dioxolan gemäss Anspruch 1 oder ein Salz davon.

6. Verfahren zur Herstellung von Verbindungen der Formel I

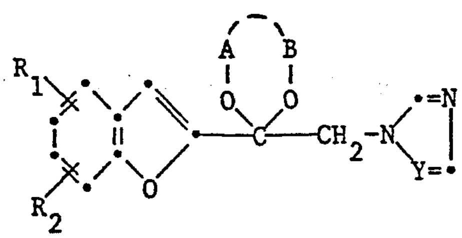 (I),

worin

Y für -CH= oder -N= steht:

$R_1$ und $R_2$ unabhängig voneinander für Wasserstoff, Halogen, $C_1$-$C_4$-Alkyl oder $C_1$-$C_4$-Alkoxy stehen;

A und B für $C_1$-$C_{12}$-Alkyl stehen oder zusammen eine der folgenden Alkylenbrücken

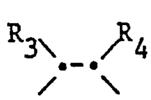 (Ia), 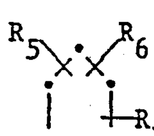 (Ib) oder 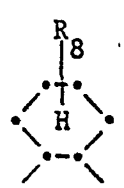 (Ic)

bedeuten, wobei

$R_3$ und $R_4$ unabhängig voneinander für Wasserstoff, ein durch Halogen oder eine Gruppe der Formel $-Z-R_9$ substituiertes oder unsubstituiertes $C_1$-$C_{12}$-Alkyl oder für ein durch Halogen oder $C_1$-$C_4$-Alkoxy und/oder $C_1$-$C_4$-Alkyl substituiertes oder unsubstituiertes Phenyl stehen, wobei Z Sauerstoff oder Schwefel bedeutet und

$R_9$ Wasserstoff, ein durch $C_1$-$C_4$-Alkoxy substituiertes oder unsubstituiertes $C_1$-$C_8$-Alkyl, $C_3$-$C_4$-Alkenyl, 2-Propinyl oder 3-Halogen-2-propinyl; ein durch Halogen, $C_1$-$C_4$-Alkyl, $C_1$-$C_4$-Alkoxy, Nitro, und/oder Trifluormethyl substituiertes oder unsubstituiertes Phenyl oder ein durch Halogen, $C_1$-$C_4$-Alkyl und/oder $C_1$-$C_4$-Alkoxy substituiertes oder unsubstituiertes Benzyl bedeutet;

$R_5$, $R_6$ und $R_7$ unabhängig voneinander Wasserstoff oder $C_1$-$C_4$-Alkyl bedeuten, wobei die Gesamtzahl der Kohlenstoffatome in $R_5$, $R_6$ und $R_7$ die Zahl 6 nicht übersteigen soll, und

$R_8$ für Wasserstoff oder $C_1$-$C_3$-Alkyl steht,

und ihrer Salze, dadurch gekennzeichnet, dass man

A) eine Verbindung der Formel II

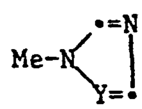

(II),

worin Me Wasserstoff oder ein Metallkation darstellt, mit einer Verbindung der Formel III

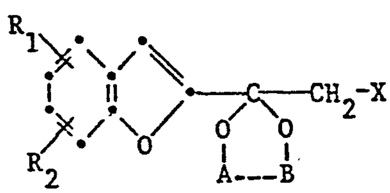

(III),

worin X eine nukleofuge Abgangsgruppe bedeutet, kondensiert oder

B) in einer Verbindung der Formel IV

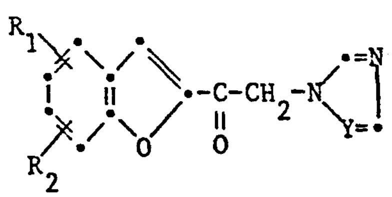

(IV).

die Carbonylgruppe in eine Gruppe der Formel V

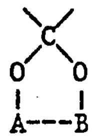

(V)

überführt, oder

C) zur Herstellung von Verbindungen der Formel I, worin A und B gemeinsam eine Gruppe der Formel $-CH_2-CH(CH_2ZR_9)-$ darstellen und $R_9$ einen von Wasserstoff verschiedenen Rest $R_9$ bedeutet, Verbindungen der Formel VI und VII

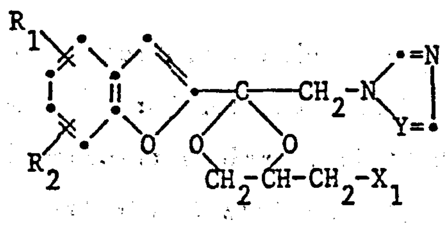

(VI) und $X_2 - R_9$ (VII),

worin einer der Reste $X_1$ und $X_2$ gegebenenfalls in Salzform vorliegendes Hydroxy oder Mercapto, z.B. der Formel -Z-Me, und der andere eine nukleofuge Abgangsgruppe X bedeutet oder sowohl $X_1$ als auch $X_2$ Hydroxygruppen darstellen, miteinander kondensiert oder

D) eine Verbindung der Formel

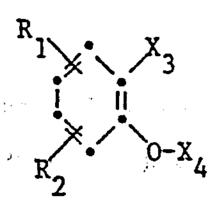

(VIII),

worin einer der Reste $X_3$ und $X_4$ eine Gruppe der Formel

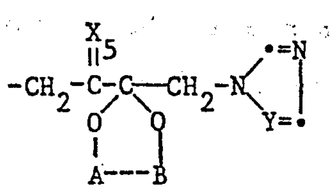

(VIIIa),

in der $X_5$ eine gegebenenfalls funktionell abgewandelte Oxogruppe ist, und der andere Wasserstoff oder $X_3$ eine Gruppe der Formel

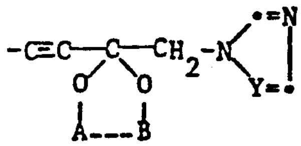

(VIIIb)

und $X_4$ Wasserstoff bedeutet, cyclisiert, oder

E) eine Verbindung der Formel

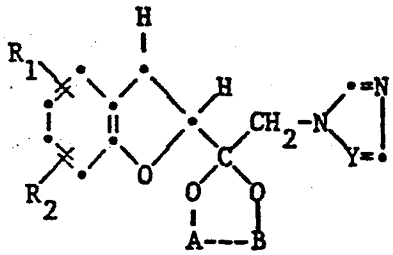

(IX)

dehydriert und, wenn erwünscht, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

7. Die nach dem Verfahren gemäss Anspruch 6 erhältlichen Verbindungen.

8. Pharmazeutische Präparate enthaltend eine oder mehrere Verbindungen gemäss einem der Ansprüche 1-7.

9. Verbindungen gemäss einem der Ansprüche 1-7 zur Anwendung in einem Verfahren zur therapeutischen Behandlung des menschlichen oder tierischen Körpers.

10. Eine Verbindung gemäss einem der Ansprüche 1-7 zur Anwendung gemäss Anspruch 9 zur Behandlung von Mykosen, Epilepsien und/oder Spannungs- und Depressionszuständen.

Patentansprüche für den Vertragsstaat AT

1. Verfahren zur Herstellung von Verbindungen der Formel I

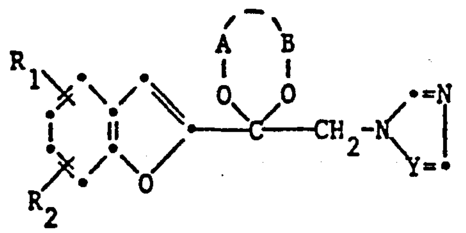

(I) ,

worin

Y für -CH= oder -N= steht:

$R_1$ und $R_2$ unabhängig voneinander für Wasserstoff, Halogen, $C_1$-$C_4$-Alkyl oder $C_1$-$C_4$-Alkoxy stehen;

A und B für $C_1$-$C_{12}$-Alkyl stehen oder zusammen eine der folgenden Alkylenbrücken

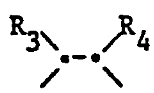 (Ia), 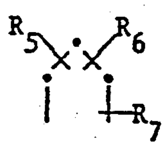 (Ib) oder 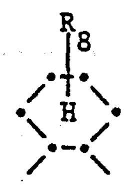 (Ic)

bedeuten, wobei

$R_3$ und $R_4$ unabhängig voneinander für Wasserstoff, ein durch Halogen oder eine Gruppe der Formel $-Z-R_9$ substituiertes oder unsubstituiertes $C_1$-$C_{12}$-Alkyl oder für ein durch Halogen, $C_1$-$C_4$-Alkoxy und/oder $C_1$-$C_4$-Alkyl substituiertes oder unsubstituiertes Phenyl stehen, wobei Z Sauerstoff oder Schwefel bedeutet und

$R_9$ Wasserstoff, ein durch $C_1$-$C_4$-Alkoxy substituiertes oder unsubstituiertes $C_1$-$C_8$-Alkyl, $C_3$-$C_4$-Alkenyl, 2-Propinyl oder 3-Halogen-2-propinyl; ein durch Halogen, $C_1$-$C_4$-Alkyl, $C_1$-$C_4$-Alkoxy, Nitro, und/oder Trifluormethyl substituiertes oder unsubstituiertes Phenyl oder ein durch Halogen, $C_1$-$C_4$-Alkyl und/oder $C_1$-$C_4$-Alkoxy substituiertes oder unsubstituiertes Benzyl bedeutet;

$R_5$, $R_6$ und $R_7$ unabhängig voneinander Wasserstoff oder $C_1$-$C_4$-Alkyl bedeuten, wobei die Gesamtzahl der Kohlenstoffatome in $R_5$, $R_6$ und $R_7$ die Zahl 6 nicht übersteigen soll, und

$R_8$ für Wasserstoff oder $C_1$-$C_3$-Alkyl steht,

und ihrer Salze, dadurch gekennzeichnet, dass man

A) eine Verbindung der Formel II

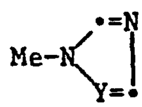

(II),

worin Me Wasserstoff oder ein Metallkation darstellt, mit einer Verbindung der Formel III

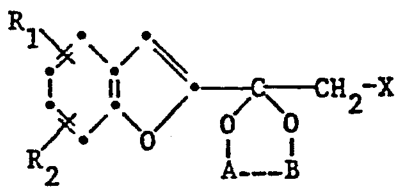

(III),

worin X eine nukleofuge Abgangsgruppe bedeutet, kondensiert oder

B) in einer Verbindung der Formel IV

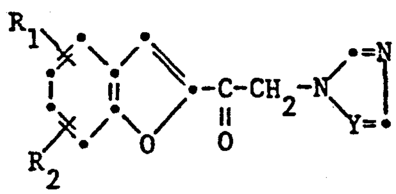

(IV)

die Carbonylgruppe in eine Gruppe der Formel V

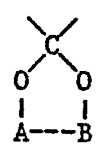

(V)

überführt, oder

C) zur Herstellung von Verbindungen der Formel I, worin A und B gemeinsam eine Gruppe der Formel $-CH_2-CH(CH_2ZR_9)-$ darstellen und $R_9$ einen von Wasserstoff verschiedenen Rest $R_9$ bedeutet, Verbindungen der Formel VI und VII

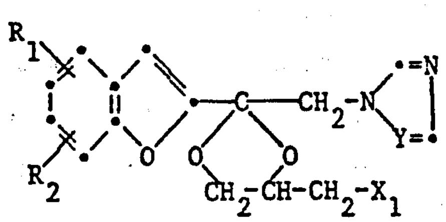 (VI) und $X_2 - R_9$ (VII),

worin einer der Reste $X_1$ und $X_2$ gegebenenfalls in Salzform vorliegendes Hydroxy oder Mercapto, z.B. der Formel -Z-Me, und der andere eine nukleofuge Abgangsgruppe X bedeutet oder sowohl $X_1$ als auch $X_2$ Hydroxygruppen darstellen, miteinander kondensiert oder

D) eine Verbindung der Formel

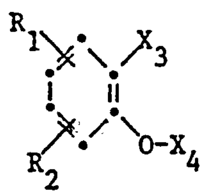 (VIII),

worin einer der Reste $X_3$ und $X_4$ eine Gruppe der Formel

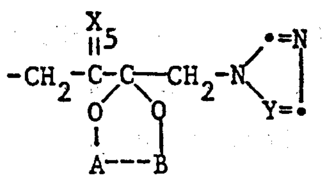 (VIIIa),

in der $X_5$ eine gegebenenfalls funktionell abgewandelte Oxogruppe ist, und der andere Wasserstoff oder $X_3$ eine Gruppe der Formel

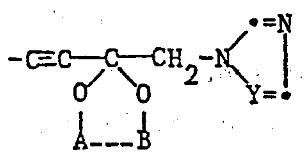 (VIIIb)

und $X_4$ Wasserstoff bedeutet, cyclisiert, oder

E) eine Verbindung der Formel

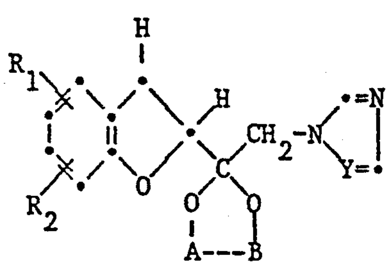 (IX)

dehydriert und, wenn erwünscht, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

2. Verfahren gemäss Anspruch 1 zur Herstellung von Verbindungen der Formel I, worin $R_1$ und $R_2$ unabhängig voneinander Wasserstoff, $C_1$-$C_4$-Alkyl, $C_1$-$C_4$-Alkoxy oder Halogen der Atomnummer bis und mit 35 bedeuten, A und B unabhängig voneinander $C_1$-$C_4$-Alkyl darstellen oder gemeinsam eine Alkylenbrücke der Formeln Ia oder Ib

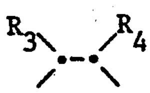 (Ia) 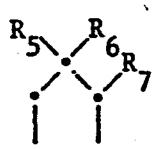 (Ib)

bilden, in der $R_3$ Wasserstoff, $C_1$-$C_4$-Alkyl oder Hydroxy-$C_1$-$C_4$-alkyl und $R_4$ Wasserstoff, $C_1$-$C_4$-Alkyl, $C_1$-$C_4$-Alkoxy-$C_1$-$C_4$-alkyl oder unsubstituiertes oder im Phenyl durch $C_1$-$C_4$-Alkyl, $C_1$-$C_4$-Alkoxy oder Halogen mit Atomnummer bis und mit 35 substituiertes Phenoxy-$C_1$-$C_4$-alkyl bedeutet bzw. $R_5$, $R_6$ und $R_7$ unabhängig voneinander Wasserstoff oder $C_1$-$C_4$-Alkyl darstellen, und Y eine Gruppe -CH= oder -N= bedeutet, und ihrer Salze.

3. Verfahren gemäss Anspruch 1 zur Herstellung von Verbindungen der Formel I, worin $R_1$ und $R_2$ entweder Wasserstoff oder Halogen der Atomnummer bis und mit 35, welches insbesondere in 5- und 7-Stellung gebunden ist, bedeuten, A und B unabhängig voneinander $C_1$-$C_4$-Alkyl darstellen oder gemeinsam eine Alkylenbrücke der Formeln Ia oder Ib

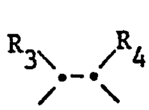 (Ia) 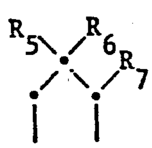 (Ib)

bilden, in der $R_3$ Wasserstoff, $C_1$-$C_4$-Alkyl oder Hydroxy-$C_1$-$C_4$-alkyl und $R_4$ Wasserstoff, $C_1$-$C_4$-Alkyl, $C_1$-$C_4$-Alkoxy-$C_1$-$C_4$-alkyl oder unsubstituiertes oder im Phenyl durch $C_1$-$C_4$-Alkyl, $C_1$-$C_4$-Alkoxy oder Halogen mit Atomnummer bis und mit 35 substituiertes Phenoxy-$C_1$-$C_4$-alkyl bedeutet bzw. $R_5$, $R_6$ und $R_7$ unabhängig voneinander Wasserstoff oder $C_1$-$C_4$-Alkyl darstellen, und Y eine Gruppe -CH= oder -N= bedeutet, und ihre Salze.

4. Verfahren gemäss Anspruch 1 zur Herstellung von
2-(Benzofuran-2-yl)-2-[1-(1H-1,2,4-triazolyl)-methyl]-1,3-dioxolan,
2-(Benzofuran-2-yl)-2-[1-(1H-1,2,4-triazolyl)-methyl]-5-ethyl-5-methyl-1,3-dioxan,
2-(Benzofuran-2-yl)-2-[1-(1H-1,2,4-triazolyl)-methyl]-5,5-dimethyl-4-propyl-1,3-dioxan,
2-(Benzofuran-2-yl)-2-[1-(1H-1,2,4-triazolyl)-methyl]-5,5-diethyl-1,3-dioxan,
2-(Benzofuran-2-yl)-2-[1-(1H-1,2,4-triazolyl)-methyl]-4-methyl-1,3-dioxan,
2-(Benzofuran-2-yl)-2-[1-(1H-1,2,4-triazolyl)-methyl]-5-ethyl-5-butyl-1,3-dioxan,
2-(Benzofuran-2-yl)-2-[1-(1H-1,2,4-triazolyl)-methyl]-5-ethyl-4-propyl-1,3-dioxan,
2-(Benzofuran-2-yl)-2-[1-(1H-1,2,4-triazolyl)-methyl]-1,3-dioxan,
2-(Benzofuran-2-yl)-2-[1-(1H-1,2,4-triazolyl)-methyl]-4-methoxymethyl-1,3-dioxolan,
2-(Benzofuran-2-yl)-2-[1-(1H-1,2,4-triazolyl)-methyl]-4,5-dimethyl-1,3-dioxolan,
2-(Benzofuran-2-yl)-2-[1-(1H-1,2,4-triazolyl)-methyl]-4-phenoxymethyl-1,3-dioxolan,

2-(Benzofuran-2-yl)-2-(1-imidazolyl-methyl)-1,3-dioxolan,
2-(Benzofuran-2-yl)-2-(1-imidazolyl-methyl)-5,5-dimethyl-1,3-dioxan,
2-(Benzofuran-2-yl)-2-(1-imidazolyl-methyl)-4-methoxymethyl-1,3-dioxolan,
2-(5,7-Dichlor-benzofuran-2-yl)-2-[1-(1H-1,2,4-triazolyl)-methyl]-4-ethyl-1,3-dioxolan,
2-(5,7-Dichlor-benzofuran-2-yl)-2-[1-(1H-1,2,4-triazolyl)-methyl]-1,3-dioxolan,
2-(5,7-Dichlor-benzofuran-2-yl)-2-[1-(1H-1,2,4-triazolyl)-methyl]-5,5-dimethyl-1,3-dioxan,
2-(5,7-Dichlor-benzofuran-2-yl)-2-[1-(1H-1,2,4-triazolyl)-methyl]-4-ethoxymethyl-1,3-dioxolan,
2-(5,7-Dichlor-benzofuran-2-yl)-2-[1-(1H-1,2,4-triazolyl)-methyl]-4-methoxymethyl-1,3-dioxolan,
2-(5,7-Diochlor-benzofuran-2-yl)-2-[1-(1H-1,2,4-triazolyl)-methyl]-5-methyl-5-propyl-1,3-dioxan,
2-(5,7-Dichlor-benzofuran-2-yl)-2-(1-imidazolylmethyl)-1,3-dioxolan,
2-(5,7-Dichlor-benzofuran-2-yl)-2-(1-imidazolylmethyl)-4-methoxymethyl-1,3-dioxolan,
2-(5,7-Dichlor-benzofuran-2-yl)-2-(1-imidazolylmethyl)-4-ethoxymethyl-1,3-dioxolan,
1-(Benzofuran-2-yl)-1,1-dimethoxy-2-[1-(1H-1,2,4-triazolyl)-ethan oder
2-(Benzofuran-2-yl)-2-[1-(1H-1,2,4-triazolyl)-methyl]-4-ethoxymethyl-1,3-dioxolan oder jeweils eines Salzes davon.

5. Verfahren gemäss Anspruch 1 zur Herstellung von 2-(5,7-Dichlor-benzofuran-2-yl)-2-[1-(1H-1,2,4-triazolyl)-methyl]-4-methyl-1,3-dioxolan oder eines Salzes davon.

6. Die nach dem Verfahren gemäss Anspruch 1 erhältlichen Verbindungen.

7. Verfahren zur Herstellung pharmazeutische Präparate, dadurch gekennzeichnet, dass man eine gemäss einem der Ansprüche 1-6 erhältliche Verbindungen mit üblichen pharmazeutischen Hilfs- und/oder Trägerstoffen vermischt.

8. Verfahren zur Herstellung pharmazeutische Präparate, dadurch gekennzeichnet, dass man

A) eine Verbindung der Formel II

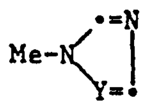

(II),

worin Me Wasserstoff oder ein Metallkation darstellt, mit einer Verbindung der Formel III

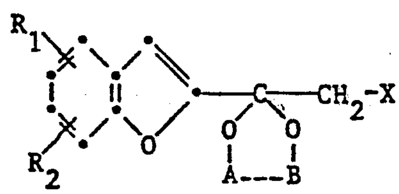

(III),

worin X eine nukleofuge Abgangsgruppe bedeutet, kondensiert oder

B) in einer Verbindung der Formel IV

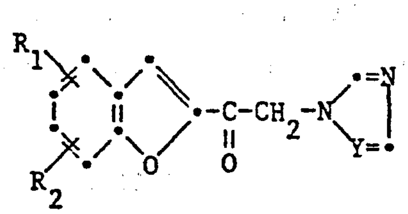

(IV)

die Carbonylgruppe in eine Gruppe der Formel V

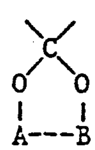

(V)

überführt, oder

C) zur Herstellung von Verbindungen der Formel I, worin A und B gemeinsam eine Gruppe der Formel $-CH_2-CH(CH_2ZR_9)-$ darstellen und $R_9$ einen von Wasserstoff verschiedenen Rest $R_9$ bedeutet, Verbindungen der Formel VI und VII

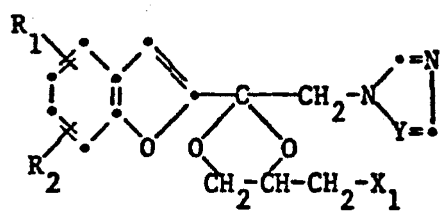 (VI) und $X_2 - R_9$. (VII),

worin einer der Reste $X_1$ und $X_2$ gegebenenfalls in Salzform vorliegendes Hydroxy oder Mercapto, z.B. der Formel -Z-Me, und der andere eine nukleofuge Abgangsgruppe X bedeutet oder sowohl $X_1$ als auch $X_2$ Hydroxygruppen darstellen, miteinander kondensiert oder

D) eine Verbindung der Formel

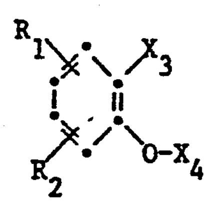 (VIII),

worin einer der Reste $X_3$ und $X_4$ eine Gruppe der Formel

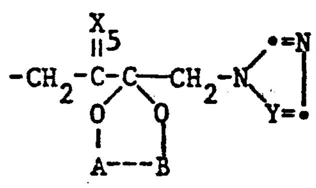 (VIIIa),

in der $X_5$ eine gegebenenfalls funktionell abgewandelte Oxogruppe ist, und der andere Wasserstoff oder $X_3$ eine Gruppe der Formel

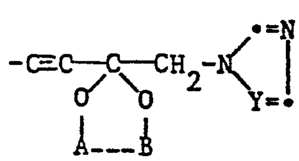 (VIIIb)

und $X_4$ Wasserstoff bedeutet, cyclisiert, oder

E) eine Verbindung der Formel

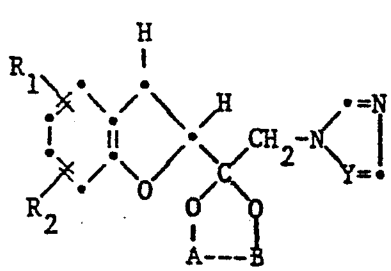 (IX)

dehydriert und, wenn erwünscht, eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt, und die verfahrensgemäss erhältliche Verbindung der Formel I

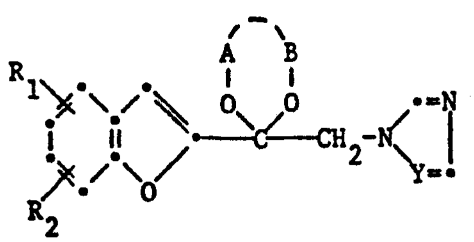

(I) ,

worin

Y für -CH= oder -N= steht;

$R_1$ und $R_2$ unabhängig voneinander für Wasserstoff, Halogen, $C_1$-$C_3$-Alkyl oder $C_1$-$C_3$-Alkoxy stehen;

A und B für $C_1$-$C_{12}$-Alkyl stehen oder zusammen eine der folgenden Alkylenbrücken

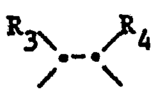 (Ia), 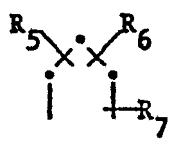 (Ib) oder 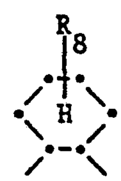 (Ic)

bedeuten, wobei

$R_3$ und $R_4$ unabhängig voneinander für Wasserstoff, ein durch Halogen oder eine Gruppe der Formel -Z-$R_9$ substituiertes oder unsubstituiertes $C_1$-$C_{12}$-Alkyl oder für ein durch Halogen, $C_1$-$C_4$-Alkoxy und/oder $C_1$-$C_4$-Alkyl substituiertes oder unsubstituiertes Phenyl stehen, wobei Z Sauerstoff oder Schwefel bedeutet und

$R_9$ Wasserstoff, ein durch $C_1$-$C_4$-Alkoxy substituiertes oder unsubstituiertes $C_1$-$C_8$-Alkyl, $C_3$-$C_4$-Alkenyl, 2-Propinyl oder 3-Halogen-2-propinyl; ein durch Halogen, $C_1$-$C_4$-Alkyl, $C_1$-$C_4$-Alkoxy, Nitro, und/oder Trifluormethyl substituiertes oder unsubstituiertes Phenyl oder ein durch Halogen, $C_1$-$C_4$-Alkyl und/oder $C_1$-$C_4$-Alkoxy substituiertes oder unsubstituiertes Benzyl bedeutet;

$R_5$, $R_6$ und $R_7$ unabhängig voneinander Wasserstoff oder $C_1$-$C_4$-Alkyl bedeuten, wobei die Gesamtzahl der Kohlenstoffatome in $R_5$, $R_6$ und $R_7$ die Zahl 6 nicht übersteigen soll, und

$R_8$ für Wasserstoff oder $C_1$-$C_3$-Alkyl steht, oder ein Salz davon mit üblichen pharmazeutischen Hilfs- und/oder Trägerstoffen vermischt.

Europäisches Patentamt

# EUROPÄISCHER RECHERCHENBERICHT

0130151

Nummer der Anmeldung

EP 84810305.7

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP - A2 - 0 065 485 (CIBA-GEIGY AG)<br>* Zusammenfassung; Ansprüche 34,36,43,44 *<br>-- | 1-10 | C 07 D 405/14<br>A 61 K 31/41<br>A 61 K 31/415 |
| A | CHEMICAL ABSTRACTS, Vol. 98, No. 11, 14. März 1983, Columbus, Ohio, USA<br>RIBALTA BARO, JOSE MIGUEL; BRUSEGHINI, LEONIDA; CASADIO, SILVANO; INIESTA PONS, JORGE. "Dioxolane amine with pharmacological activity." Seite 558, Spalte 2, Abstract No. 89344j<br>& Span. ES 505 979<br>-- | 1 | |
| A | CHEMICAL ABSTRACTS, Vol. 98, No. 11, 14. März 1983, Columbus, Ohio, USA<br>RIBALTA BARO, JOSE MIGUEL; BRUSEGHINI, LEONIDA; CASADIO, SILVANO; INIESTA PONS, JORGE. "Benzofuranyldioxolanes with pharmacological activity." Seite 558, Spalte 2, Abstract No. 89345k<br>& Span. ES 506 904<br>-- | 1 | RECHERCHIERTE SACHGEBIETE (Int. Cl. 3)<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 29-08-1984 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

0130151
Nummer der Anmeldung

EP 84810305.7

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| A | CHEMICAL ABSTRACTS, Vol. 98, No. 15, 11. April 1983, Columbus, Ohio, USA<br>CIBA-GEIGY A.-G. "Ketals and microbiocides containing same." Seite 642, Spalte 2, Abstract No. 126097d<br>& Jpn. Kokai Tokkyo Koho JP 57,122,083 [82,122,083]<br>---- | 1 | |
| | | | RECHERCHIERTE SACHGEBIETE (Int. Cl.³) |

EPA Form 1503.2 06.78